# EUROPEAN PATENT APPLICATION

(11) **EP 4 428 127 A1**
(43) Date of publication of application: **11.09.2024**
(21) Application number: 22888765.9
(22) Date of filing: 14.04.2022
(51) Int. Cl.: C07D 263/62, C07D 277/82, C07D 413/14, C07D 413/12, C07D 417/12, C07D 417/14, C09K 11/00, H10K 50/80

(54) **ORGANIC COMPOUND, LIGHT EXTRACTION LAYER MATERIAL, AND ORGANIC ELECTRONIC DEVICE**

(30) Priority: 03.11.2021 CN 202111292496
(71) Applicant: Guangzhou Chinaray Optoelectronic Materials Ltd., Guangzhou, Guangdong 510663 (CN)
(72) Inventor: LI, Tao, Guangzhou, Guangdong 510663 (CN); LONG, Zhijun, Guangzhou, Guangdong 510663 (CN); SONG, Jingyao, Guangzhou, Guangdong 510663 (CN)
(74) Representative: Nederlandsch Octrooibureau
(86) International application number: PCT/CN2022/086765
(87) International publication number: WO 2023/077726

(57) **Abstract**

The present disclosure provides an organic compound, a light extraction layer material, and an organic electronic device. The organic compound has a structure represented by formula (1). The organic compound can be used in organic electroluminescent devices as a light extraction layer material to improve light-extracting efficiency of the devices.

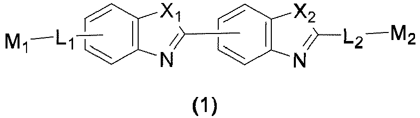

## Description

### TECHNICAL FIELD

The present disclosure relates to the field of organic electroluminescence, and in particular, to organic compounds, light extraction layer materials, and organic electronic devices.

### BACKGROUND

Organic electroluminescent display devices are a self-luminescence type of display device that generates excitons through the transfer and recombination of charge carriers between different functional layers and emits light relying on high quantum efficiency of organic compounds or metallic coordination compounds, and has characteristics of self-luminescence, high brightness, high efficiency, high contrast, high responsiveness, and the like.

In recent years, luminescence efficiency of organic light-emitting diodes (OLEDs) has been greatly improved, but their internal quantum efficiency is close to the theoretical limit. Therefore, improving the light-extracting efficiency of OLEDs has become an effective means to further improve stability and current efficiency of devices, such as the accumulation of metallic coordination compounds in emitting layers and the matching of refractive index between various functional layers.

OLEDs are light-emitting devices with top-light emitting structures. For such light-emitting devices, when the light emitted by a light-emitting layer incidents into other films at a certain angle or above, it will be fully reflected at the interface between the light-emitting layer and other films, causing the light emitted by the light-emitting layer can not be emitted out of the devices, resulting in only a portion of the emitted light being utilized. In recent years, in order to improve light-extracting efficiency, traditional technologies have adopted light-emitting devices with high refractive index of "covering layers" disposed outside the semi-transparent electrodes with low refractive index. For example, in 2003, Riel et al. deposited ZnSe, an inorganic compound with high refractive index (n=2.6), on the cathode, and improved light-extracting efficiency by utilizing the refractive index difference between functional layers. However, due to the high evaporation temperature and slow evaporation rate of inorganic materials, the compound has not been widely used in organic electroluminescent devices.

### TECHNICAL PROBLEMS

Based on the above, the present disclosure provides an organic compound that can be used as a light extraction layer material of organic electroluminescent devices, thereby improving light-extracting efficiency of devices.

### TECHNICAL SOLUTIONS

The present disclosure is embodied through the following technical solutions:
An organic compound having a structure represented by formula (1): 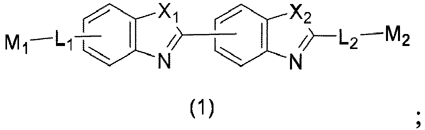 wherein,
X₁ and X₂ are independently selected from O, S, or NR₁;
L₁ and L₂ are independently selected from a single bond, a substituted or unsubstituted aromatic group having 6 to 30 ring atoms, or a substituted or unsubstituted heteroaromatic group having 5 to 30 ring atoms;
M₁ and M₂ are independently selected from a substituted or unsubstituted aromatic group having 6 to 60 ring atoms, a substituted or unsubstituted heteroaromatic group having 5 to 60 ring atoms, or a substituted or unsubstituted amino group; and
R₁ at each occurrence is independently selected from -H, -D, a linear alkyl group having 1 to 20 carbon atoms, a branched alkyl group having 3 to 20 carbon atoms, an alkenyl group having 2 to 20 carbon atoms, a cyclic alkyl group having 3 to 20 carbon atoms, a substituted or unsubstituted aromatic group having 6 to 30 ring atoms, or a substituted or unsubstituted heteroaromatic group having 5 to 30 ring atoms.

The present disclosure further provides a mixture including an organic compound as described above and at least one organic functional material. The organic functional material is selected from a hole injection material, a hole transport material, an electron transport material, an electron injection material, an electron blocking material, a hole blocking material, an emitter, or a host material.

The present disclosure further provides a composition including the organic compound or the mixture as described above, and at least one organic solvent.

The present disclosure further provides a light extraction layer material. The light extraction layer material includes the organic compound or the mixture as described above, or is prepared from the above-mentioned composition.

The present disclosure further provides an organic electronic device including the organic compound or the mixture as described above, or prepared from the above-mentioned.

### BENEFICIAL EFFECTS

Compared with the prior art, the organic compound of the present disclosure has the following beneficial effects:
The organic compound of the present disclosure has higher absorption to the light in the ultraviolet spectrum, that is, a higher extinction coefficient in the ultraviolet spectrum (<400 nm), which can resist the damage of external light with high-energy to components in devices, thus avoiding unfavorable effects of harmful light on materials in devices. Moreover, the organic compound has weaker absorption to the light in the visible spectrum, reflected in the extinction coefficient close to 0 in the range of the visible light (>430 nm), making the organic compound have higher transmissivity to visible light, higher refractive index, and smaller refractive index differences in the range of the visible light, thus ensuring better light-extracting effects. Meanwhile, the organic compound of the present disclosure also has a high glass transition temperature and a high thermal decomposition temperature.

### BRIEF DESCRIPTION OF THE DRAWINGS

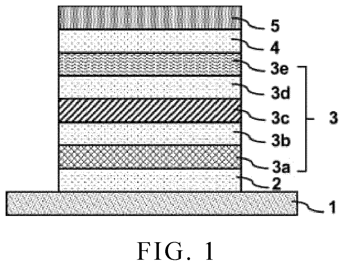

FIG. 1 is a schematic structural diagram of a light-emitting device provided by an embodiment of the present disclosure. In the figure, 1 indicates a substrate, 2 indicates an anode, 3a indicates a hole injection layer, 3b indicates a hole transport layer, 3c indicates a light-emitting layer, 3d indicates an electron transport layer, 3e indicates an electron injection layer, 4 indicates a cathode, and 5 indicates a light-extracting layer.

### DETAILED DESCRIPTION

For the convenience of understanding the present disclosure, a more comprehensive description will be provided below. Preferable embodiments of the present disclosure will be provided. This disclosure may, however, be embodied in different forms and should not be construed as limitation to the embodiments set forth herein. Rather, these embodiments are provided so that the present disclosure will be thorough and complete.

Unless otherwise defined, all terms, including technical and scientific terms, used herein have the same meaning as those commonly understood by one of ordinary skill in the art to which the present disclosure belongs. The terms used in the specification of the present disclosure are only for the purpose of describing specific embodiments and are not intended to limit the present disclosure. The term "and/or" used in this context includes any and all combinations of one or more of the associated listed items.

In the present disclosure, a composition, a printing ink, and an ink have the same meaning and may be interchanged.

In the present disclosure, an aromatic group and an aromatic ring system have the same meaning and may be interchanged.

In the present disclosure, a heteroaromatic group and a heteroaromatic ring system have the same meaning and may be interchanged.

In the present disclosure, "substituted" means that a hydrogen atom in a group to be substituted is substituted by a substituent group R.

In the present disclosure, the same substituent groups at different substituent sites may be independently selected from different groups. For example, if a formula includes multiple R₁ groups, the R₁ groups may be independently selected from different groups.

In the present disclosure, "substituted or unsubstituted" means that a group to be defined, i.e., a defined group, may be substituted or not be substituted. When the defined group is substituted, it can be understood that the defined group may be substituted by one or more substituent R groups. The R groups are independently selected from, but not limited to, deuterium atom (D), a cyano group, an isocyano group, a nitro group, a halogen group, an alkyl group having 1 to 20 carbon atoms, a heterocyclic group having 3 to 20 ring atoms, an aromatic group having 6 to 20 ring atoms, a heteroaromatic group having 5 to 20 ring atoms, -NR'R", a silyl group, a carbonyl group, an alkoxycarbonyl group, an aryloxycarbonyl group, an aminoformyl group, a haloformyl group, a formyl group, an isocyanate group, a thiocyanate group, an isothiocyanate group, a hydroxyl group, or a trifluoromethyl group, and the groups may further be substituted by suitable substituent groups in the art. Understandably, R' and R" in the NR'R" are independently selected from, but not limited to, H, D, a cyano group, an isocyano group, a nitro group, a halogen group, an alkyl group having 1 to 10 carbon atoms, a heterocyclic group having 3 to 20 ring atoms, an aromatic group having 6 to 20 ring atoms, a heteroaromatic group having 5 to 20 ring atoms. Preferably, R is selected from, but not limited to, D, a cyano group, an isocyano group, a nitro group, a halogen group, an alkyl group having 1 to 10 carbon atoms, a heterocyclic group having 3 to 10 ring atoms, an aromatic group having 6 to 20 ring atoms, a heteroaromatic group having 5 to 20 ring atoms, a silyl group, a carbonyl group, an alkoxycarbonyl group, an aryloxycarbonyl group, an aminoformyl group, a haloformyl group, a formyl group, an isocyanate group, a thiocyanate group, an isothiocyanate group, a hydroxyl group, or a trifluoromethyl group, and the groups may further be substituted by suitable substituent groups in the art.

In the present disclosure, "the number of ring atoms" refers to the number of atoms constituting a ring in a structural compound (for example, a monocyclic compound, a fused ring compound, a cross-linked compound, a carbon ring compound, or a heterocyclic compound) obtained by atomic bonding. In a ring substituted by a substituent group, the atoms contained in the substituent group are not included in the atoms forming the ring. The same applies to "the number of ring atoms" described below unless otherwise specified. For example, the number of ring atoms in a benzene ring is 6, the number of ring atoms in a naphthalene ring is 10, and the number of ring atoms in thienyl is 5.

"An aryl group or an aromatic group" refers to an aromatic hydrocarbon group derived from a basis of an aromatic ring compound removing one hydrogen atom. The aromatic hydrocarbon group may be an aryl group with a single ring, a fused ring aryl group, or a polycyclic aryl group. For a polycyclic ring type, at least one ring is an aromatic ring system. For example, "a substituted or unsubstituted aryl group having 6 to 40 ring atoms" refers to an aryl group having 6 to 40 ring atoms, preferably a substituted or unsubstituted aryl group having 6 to 30 ring atoms, more preferably a substituted or unsubstituted aryl group having 6 to 18 ring atoms, most preferably a substituted or unsubstituted aryl group having 6 to 14 ring atoms, and the aryl group is optionally further substituted. Suitable examples of the aryl group or the aromatic group include, but not limited to, a phenyl group, a biphenyl group, a triphenyl group, a naphthyl group, an anthracyl group, a phenanthryl group, a fluoranthenyl group, a triphenylene group, a pyrenyl group, a perylene group, a tetraphenyl group, a fluorenyl group, a diphenyl group, an acenaphthenyl group, and derivatives thereof. Understandably, multiple aryl groups may further be disconnected by short non-aromatic units (for example, a non-hydrogenium atom contenting less than 10%, such as C, N, or O). In particular, an acenaphthene group, a fluorene group, a 9,9-diarylfluorene group, a triarylamine group, or a diaryl ether system may be further included in a definition of the aryl group.

"A heteroaryl group or a heteroaromatic group" refers to a basis of an aryl group with at least one carbon atom substituted by a non-carbon atom, and the non-carbon atom may be N, O, S, or the like. For example, "a substituted or unsubstituted heteroaryl group having 5 to 40 ring atoms" refers to a heteroaryl group having 5 to 40 ring atoms, preferably a substituted or unsubstituted heteroaryl group having 6 to 30 ring atoms, more preferably a substituted or unsubstituted heteroaryl group having 6 to 18 ring atoms, most preferably a substituted or unsubstituted heteroaryl group having 6 to 14 ring atoms, and the heteroaryl group is optionally further substituted. Suitable examples of the heteroaryl group or the heteroaromatic group include, but not limited to, a thienyl group, a furyl group, a pyrrolyl group, a diazole group, a triazole group, an imidazolyl group, a pyridinyl group, a bipyridyl group, a pyrimidinyl group, a triazinyl group, an acridinyl group, a pyridazinyl group, a pyrazinyl group, a quinolinyl group, an isoquinolinyl group, a quinazolinyl group, a quinoxalinyl group, a phthalazinyl group, a pyridino pyrimidinyl group, a pyridino pyrazinyl group, a benzothiophenyl group, a benzofuranyl group, an indolyl group, a pyrrolo imidazolyl group, a pyrrolopyrrolyl group, a thiophenopyrrolyl group, a thiophenothiophenyl group, a furanopyrrolyl group, a furanofuranyl group, a thiophenofuranyl group, a benzoisoxazolyl group, a benzoisothiazolyl group, a benzimidazolyl group, an o-diazonaphthalyl group, a phenanthridinyl group, a berberine group, a quinazolinketone group, a dibenzothiophenyl group, a dibenzofuranyl group, a carbazolyl group, and derivatives thereof.

In the present disclosure, "an alkyl group" refers to a linear alkyl group, a branched alkyl group, and/or a cyclic alkyl group. The number of carbon atoms in the alkyl group may range from 1 to 50, 1 to 30, 1 to 20, 1 to 10, or 1 to 6. The term containing the alkyl group, such as "a C₁₋₉ alkyl group", refers to an alkyl group containing 1 to 9 carbon atoms. The C₁₋₉ alkyl group at each occurrence is independently selected from a C₁ alkyl group, a C₂ alkyl group, a C₃ alkyl group, a C₄ alkyl group, a C₅ alkyl group, a C₆ alkyl group, a C₇ alkyl group, a C₈ alkyl group, or a C₉ alkyl group. Examples of the alkyl group include, but not limited to, methyl, ethyl, n-propyl, isopropyl, n-butyl, sec-butyl, tert-butyl, isobutyl, 2-ethylbutyl, 3,3-dimethylbutyl, n-pentyl, isopentyl, neopentyl, tert-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butyhexyl, cyclohexyl, 4-methylcyclohexyl, 4-tert-butylcyclohexyl, n-heptyl, 1-methylheptyl, 2,2-dimethylheptyl, 2-ethylheptyl, 2-butyl-heptyl, n-octyl, tert-octyl, 2-ethyloctyl, 2-butyl-octyl, 2-hexyl-octyl, 3,7-dimethyloctyl, cyclooctyl, n-nonyl, n-decanyl, adamantine, 2-ethyldecyl, 2-butyldecyl, 2-hexyldecyl, 2-octyldecyl, n-undecyl, n-dodecyl, 2-ethyldodecyl, 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyeicosyl, 2-hexyleicosyl, 2-octyleicosyl, n-heneicosyl, n-docosyl, n-tricosyl, n-tetracosyl, n-pentacosyl, n-hexacosyl, n-heptacosyl, n-octacosyl, n-nonacosyl, n-triacontyl, and the like.

In the present disclosure, substituent groups and abbreviations thereof are as follows: normal-n, secondary-sec, iso-i, tertiary-t, ortho-o, meta-m, para-p, methyl-Me, ethyl-Et, propyl-Pr, butyl-Bu, n-amyl-Am, hexyl-Hx, and cyclohexyl-Cy.

"An amino group" refers to a derivative of amine represented by formula -N(X)₂, in which "X" is independently selected from H, a substituted or unsubstituted alkyl group, a substituted or unsubstituted cycloalkyl group, a substituted or unsubstituted heterocyclic group, or the like. Examples of the amino group include, but not limited to, -NH₂, -N-(alkyl group)₂, -NH-(alkyl group), -N-(cycloalkyl group)₂, -NH-(cycloalkyl group), -N-(heterocyclic group)₂, -NH-(heterocyclic group), -N-(aryl group)₂, -NH-(aryl group), -N-(alkyl group)(aryl group), -N-(alkyl group)(heterocyclic group), -N-(cycloalkyl group)(heterocyclic group), -N-(aryl group)(heteroaryl group), -N-(alkyl group)(heteroaryl group), and the like.

In the present disclosure, "*" connected to a single bond indicates a linking site or a fusing site.

In the present disclosure, when a linking site in a group is not specified, it means that any of connectable sites in the group may be selected as the linking site.

In the present disclosure, when a fusing site in a group is not specified, it means that any of fusible sites in the group may be selected as the fusing site. Preferably, two or more adjacent sites in the group form a fusing site.

In the present disclosure, when there are more than one substituent group having the same symbol on the same group, the substituent groups may be the same or different. For example, in formula 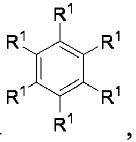 six R¹ groups in the benzene ring may be the same or different.

In the present disclosure, a single bond connected to a substituent group and penetrated through a corresponding ring indicates that the substituent group may be connected to any site of the ring. For example, 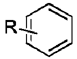 means that R may be connected to any substituent site in the benzene ring, and 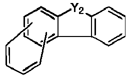 means that 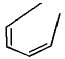 may be connected to any substituent sites in the benzene ring of 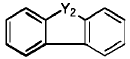 to form a union ring.

According to the present disclosure, a cyclic alkyl group and a cycloalkyl group have the same meaning and may be interchanged.

In the present disclosure, "adjacent groups" refer to two groups absent of substitutable sites between the two groups.

The present disclosure provides an organic compound having a structure represented by formula (1): 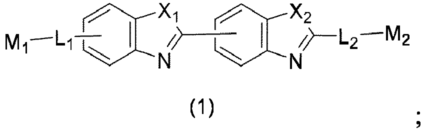 wherein,
X₁ and X₂ are independently selected from O, S, or NR₁;
L₁ and L₂ are independently selected from a single bond, a substituted or unsubstituted aromatic group having 6 to 30 ring atoms, or a substituted or unsubstituted heteroaromatic group having 5 to 30 ring atoms;
M₁ and M₂ are independently selected from a substituted or unsubstituted aromatic group having 6 to 60 ring atoms, a substituted or unsubstituted heteroaromatic group having 5 to 60 ring atoms, or a substituted or unsubstituted amino group; and
R₁ at each occurrence is independently selected from -H, -D, a linear alkyl group having 1 to 20 carbon atoms, a branched alkyl group having 3 to 20 carbon atoms, an alkenyl group having 2 to 20 carbon atoms, a cyclic alkyl group having 3 to 20 carbon atoms, a substituted or unsubstituted aromatic group having 6 to 30 ring atoms, or a substituted or unsubstituted heteroaromatic group having 5 to 30 ring atoms.

In one specific embodiment, the substituent group R in "a substituted or unsubstituted group" is preferably selected from an alkyl group having 1 to 10 carbon atoms, an aryl group having 6 to 10 ring atoms, or a heteroaryl group having 6 to 10 ring atoms.

In one embodiment, R₁ at each occurrence is independently selected from -H, -D, a linear alkyl group having 1 to 10 carbon atoms, a branched alkyl group having 3 to 10 carbon atoms, a cyclic alkyl group having 3 to 10 carbon atoms, a phenyl group, a biphenyl group, a triphenyl group, a pyrimidinyl group, a pyrimidinyl group, a triazinyl group, a naphthyl group, a quinolyl group, or an isoquinolyl group.

In one specific embodiment, X₁ and X₂ are independently selected from O or S.

Further, the organic compound has a structure represented by formula (2-1) or formula (2-2):

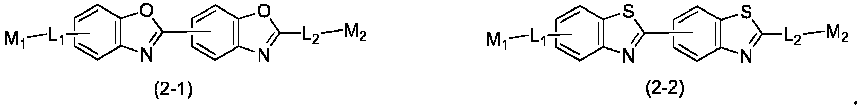

In one embodiment, 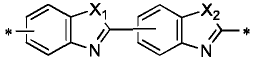 is selected from a group consisting of following structures: 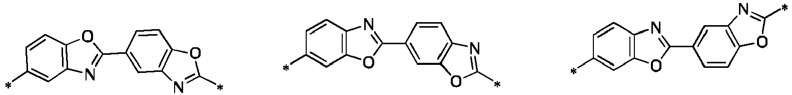 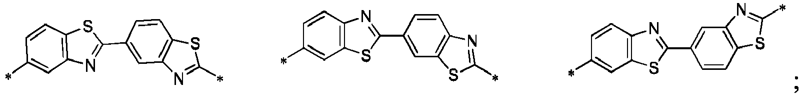 in which * indicates a linking site.

In one specific embodiment, L₁ and L₂ are independently selected from a single bond, a substituted or unsubstituted aromatic group having 6 to 20 ring atoms, or a substituted or unsubstituted heteroaromatic group having 5 to 20 ring atoms.

In one specific embodiment, L₁ and L₂ are independently selected from a single bond and a group consisting of following groups: 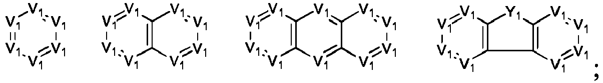 wherein,
V₁ at each occurrence is independently selected from CR₂ or N;
Y₁ is selected from NR₃, CR₄R₅, SiR₄R₅, O, S, S=O, or SO₂; and
R₂, R₃, R₄, and R₅ at each occurrence are independently selected from -H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 5 to 20 ring atoms, a substituted or unsubstituted aryloxy group having 5 to 20 ring atoms, a substituted or unsubstituted heteroaryloxy group having 5 to 20 ring atoms, or any combination of these groups.

Understandably, in the present disclosure, when V₁ is a linking site, V₁ is C.

Further, L₁ and L₂ are independently selected from a single bond, a phenyl group, or a carbazolyl group.

In another embodiment, both of L₁ and L₂ are a single bond.

In one specific embodiment, M₁ and M₂ are independently selected from a substituted or unsubstituted aromatic group having 6 to 30 ring atoms, a substituted or unsubstituted heteroaromatic group having 5 to 30 ring atoms, or a substituted or unsubstituted amino group.

In one specific embodiment, M₁ and M₂ are independently selected from a group consisting of following groups: 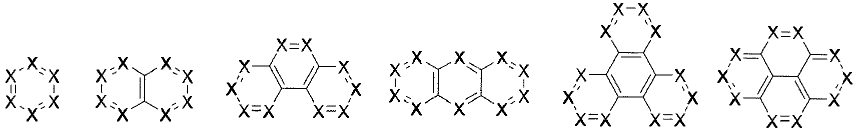 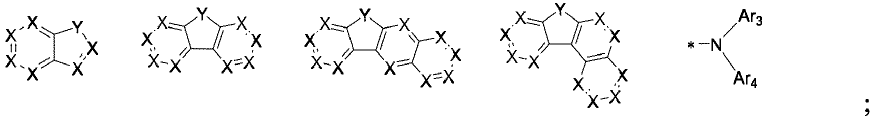 wherein,
X at each occurrence is independently selected from CR₆ or N;
Y at each occurrence is selected from NR₇, CR₈R₉, SiR₈R₉, O, S, S=O, or SO₂;
R₆, R₇, R₈, and R₉ at each occurrence are independently selected from-H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 30 ring atoms, a substituted or unsubstituted heteroaromatic group having 5 to 30 ring atoms, a substituted or unsubstituted aryloxy group having 5 to 30 ring atoms, a substituted or unsubstituted heteroaryloxy group having 5 to 30 ring atoms, or any combination of these groups; and R₈ and R₉ form a ring or do not form a ring together;
Ar₃ and Ar₄ are independently selected from a substituted or unsubstituted aromatic group having 6 to 30 ring atoms or a substituted or unsubstituted heteroaromatic group having 5 to 30 ring atoms; and
the symbol "*" indicates a linking site.

Understandably, in the present disclosure, when X is a linking site, X is C.

In one embodiment, R₆, R₇, R₈, and R₉ at each occurrence are independently selected from -H, -D, a linear alkyl group having 1 to 10 carbon atoms, a branched alkyl group having 3 to 10 carbon atoms, a cyclic alkyl group having 3 to 10 carbon atoms, a silyl group, a cyano group, an isocyano group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 6 to 20 ring atoms, or any combination of these groups.

In one embodiment, R₆, R₇, R₈, and R₉ at each occurrence are independently selected from -H, -D, a linear alkyl group having 1 to 4 carbon atoms, a branched alkyl group having 3 to 4 carbon atoms, a cyclic alkyl group having 3 to 10 carbon atoms, a silyl group, a cyano group, an isocyano group, a hydroxyl group, a nitro group, an amino group, -CF₃, -Cl, -Br, -F, -I, a substituted or unsubstituted aromatic group having 6 to 10 ring atoms, a substituted or unsubstituted heteroaromatic group having 6 to 13 ring atoms, or any combination of these groups.

In one specific embodiment, Ar₃ and Ar₄ are independently selected from a group consisting of following groups: 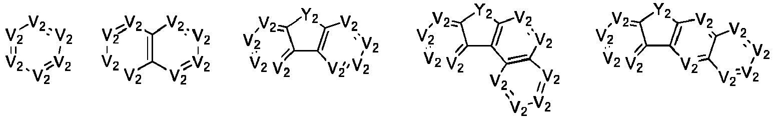 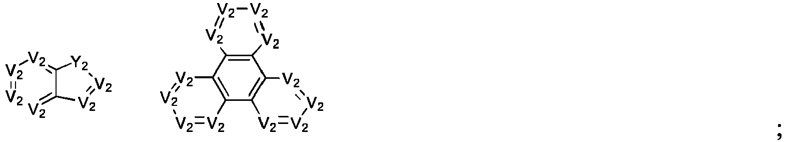 wherein,
V₂ at each occurrence is independently selected from CR₁₀ or N;
Y₂ is selected from NR₁₁, CR₁₂R₁₃, SiR₁₂R₁₃, O, S, S=O, or SO₂; and
R₁₀, R₁₁, R₁₂, and R₁₃ at each occurrence are independently selected from -H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 5 to 20 ring atoms, a substituted or unsubstituted aryloxy group having 5 to 20 ring atoms, a substituted or unsubstituted heteroaryloxy group having 5 to 20 ring atoms, or any combination of these groups; and R₁₂ and R₁₃ form a ring or do not form a ring together.

When V₂ is a linking site, V₂ is selected from C.

In one embodiment, R₁₀, R₁₁, R₁₂, and R₁₃ at each occurrence are independently selected from -H, -D, a linear alkyl group having 1 to 10 carbon atoms, a branched alkyl group having 3 to 10 carbon atoms, a cyclic alkyl group having 3 to 10 carbon atoms, a silyl group, a cyano group, an isocyano group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 6 to 20 ring atoms, or any combination of these groups.

In one embodiment, R₁₀, R₁₁, R₁₂, and R₁₃ at each occurrence are independently selected from -H, -D, a linear alkyl group having 1 to 4 carbon atoms, a branched alkyl group having 3 to 4 carbon atoms, a cyclic alkyl group having 3 to 10 carbon atoms, a silyl group, a cyano group, an isocyano group, a hydroxyl group, a nitro group, an amino group, -CF₃, -Cl, -Br, -F, -I, a substituted or unsubstituted aromatic group having 6 to 10 ring atoms, a substituted or unsubstituted heteroaromatic group having 6 to 13 ring atoms, or any combination of these groups.

Preferably, M₁ and M₂ are independently selected from a substituted or unsubstituted amino group or a substituted or unsubstituted heteroaromatic group having 9 to 30 ring atoms, in which the high electron density and molar refractive index of heteroatoms can increase the refractive index of molecules and regulate absorption ranges of molecules, thus enhancing absorption intensity in the range of ultraviolet spectrum.

More preferably, the heteroaromatic group is selected from 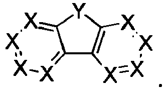 The structure has a large conjugated structure and high planarity, which can enhance the thermal properties of molecules containing the group, such as Tg and Td.

In one specific embodiment, at least one of M₁ and M₂ is selected from 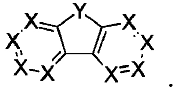 Further, 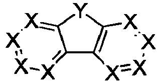 is selected from 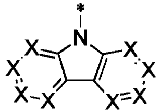 or

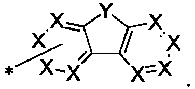

In one specific embodiment, the organic compound has a structure represented by any one of formulae (3-1) to (3-7):

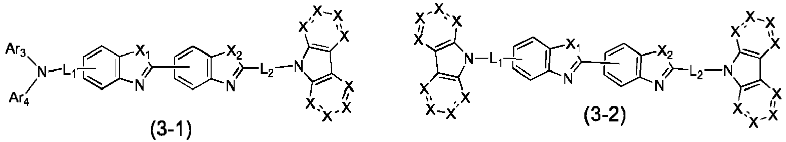

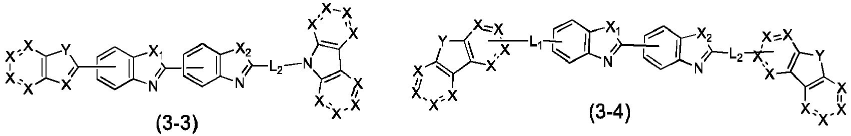

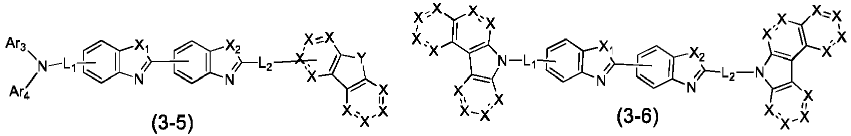

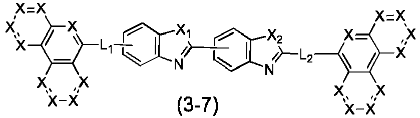

In one specific embodiment, M₁ and M₂ are independently selected from a group consisting of following groups: 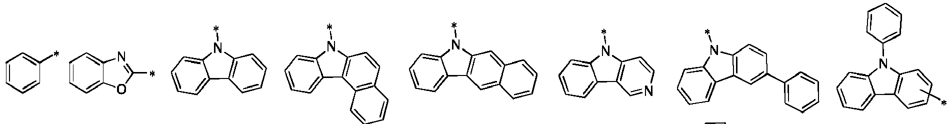 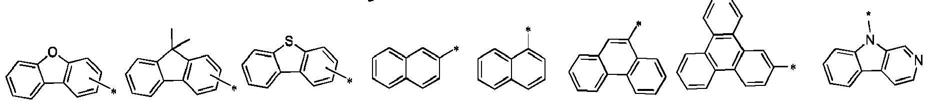 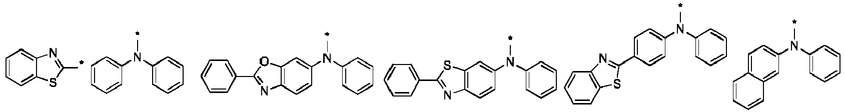 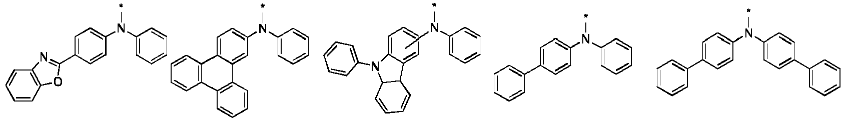 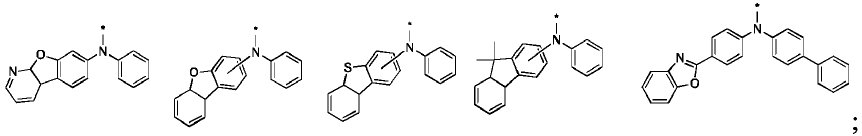 in which * indicates a linking site.

In one embodiment, both of L₁ and L₂ in the formulae (3-1) to (3-7) are a single bond

The organic compound of the present disclosure is selected from, but not limited to, a group consisting of following structure:

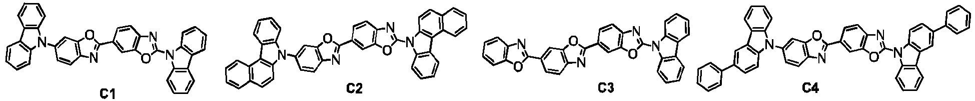

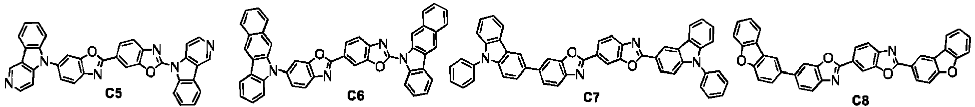

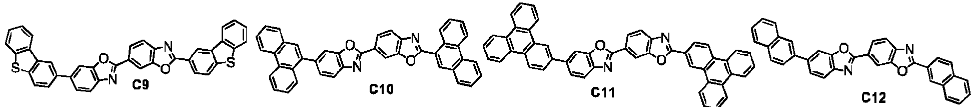

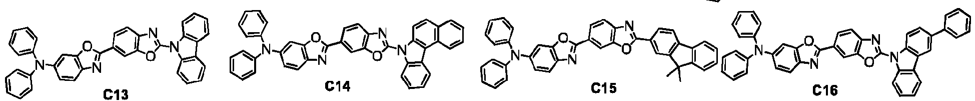

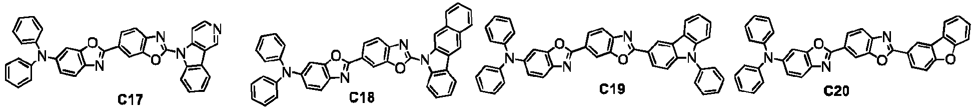

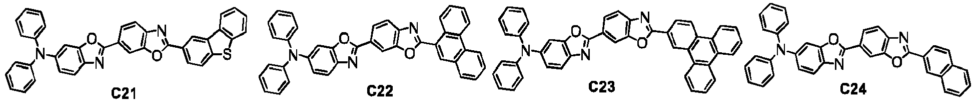

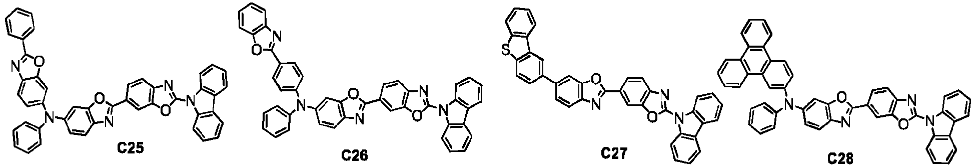

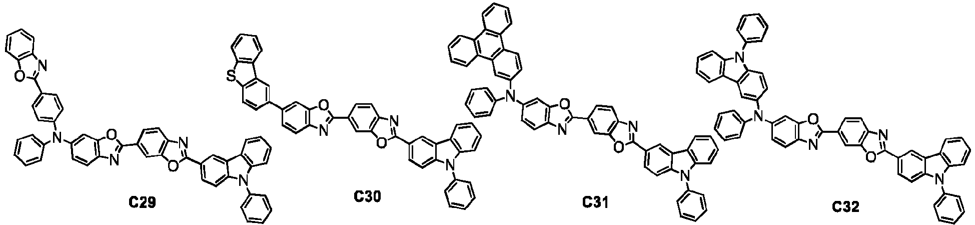

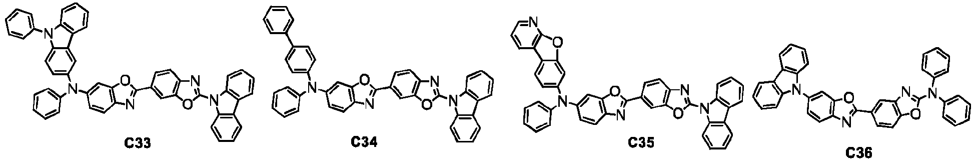

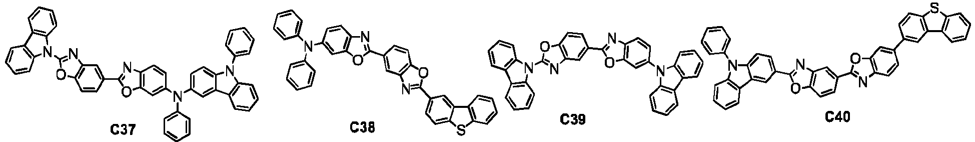

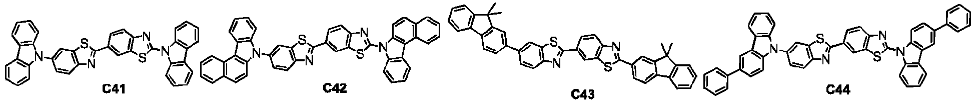

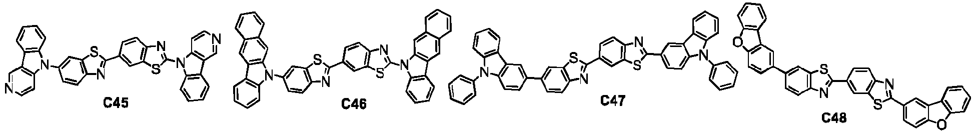

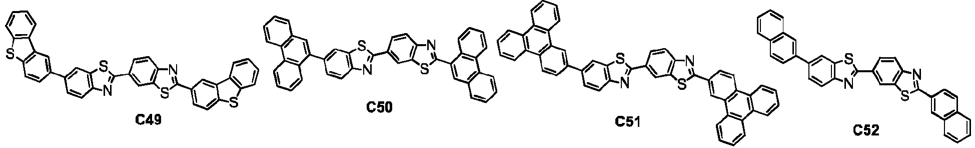

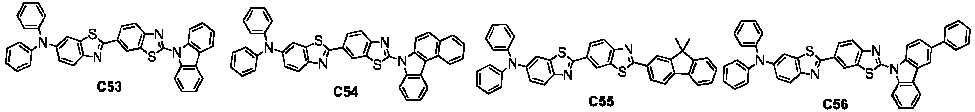

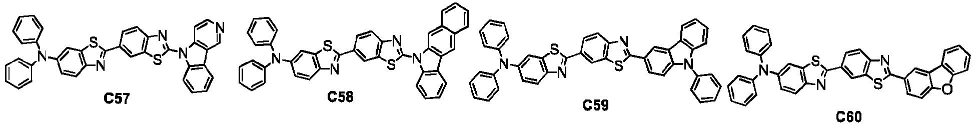

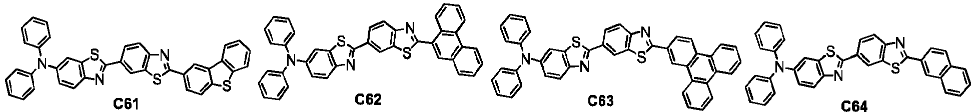

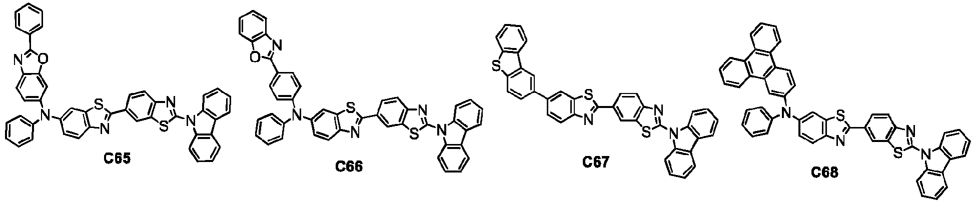

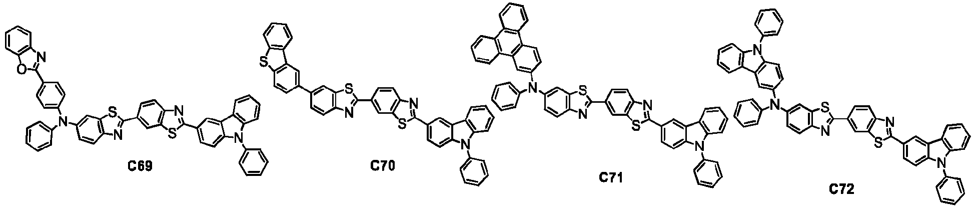

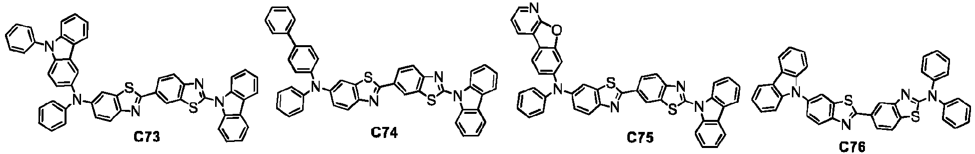

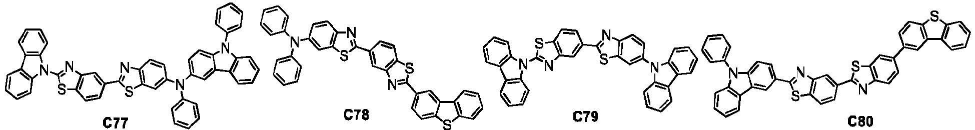

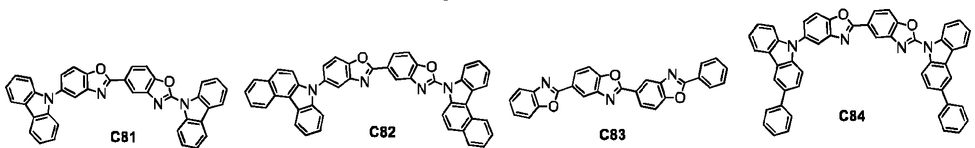

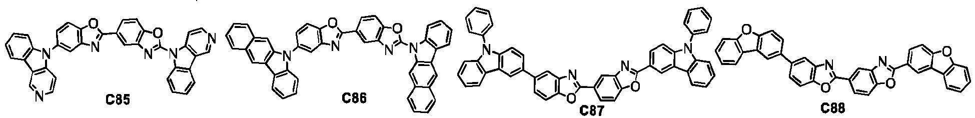

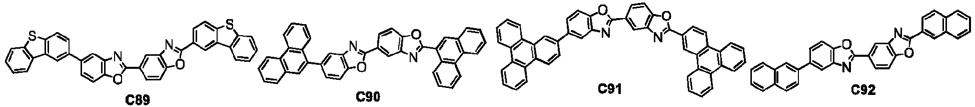

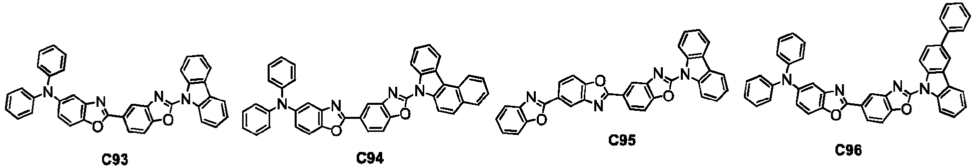

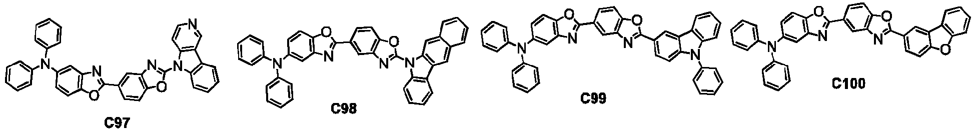

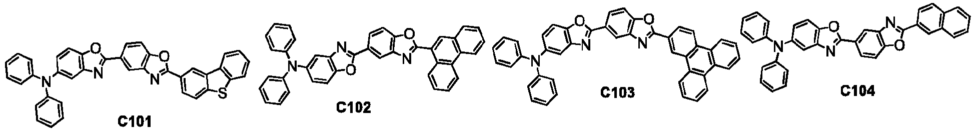

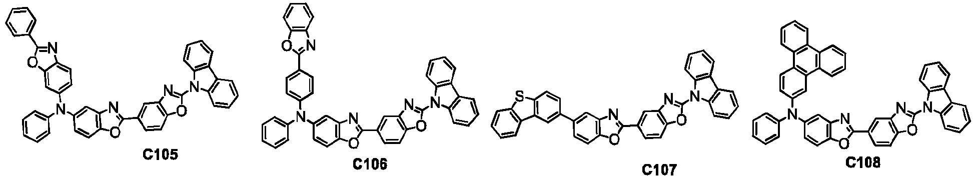

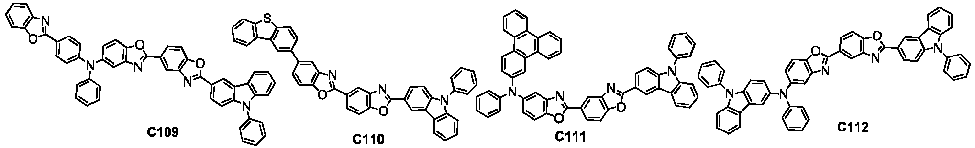

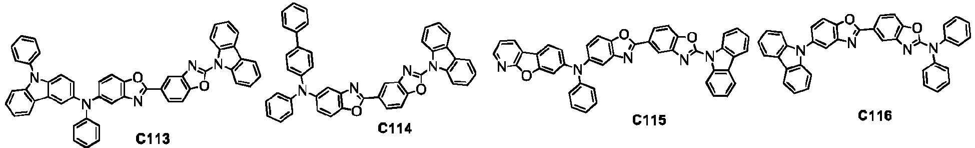

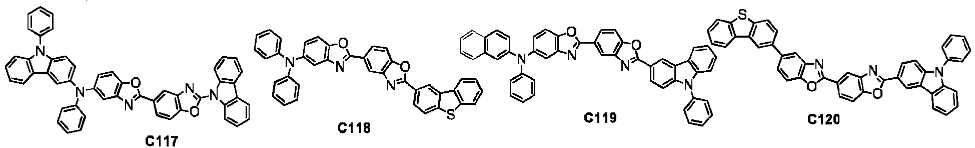

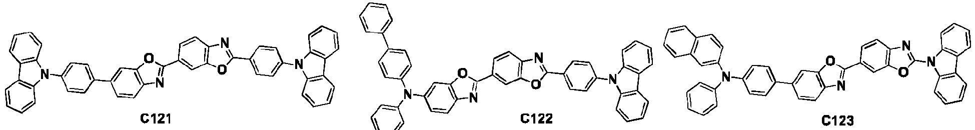

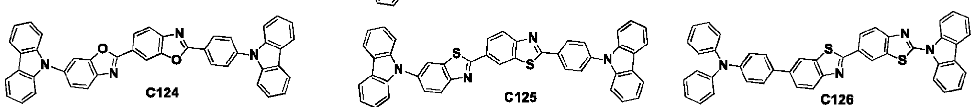

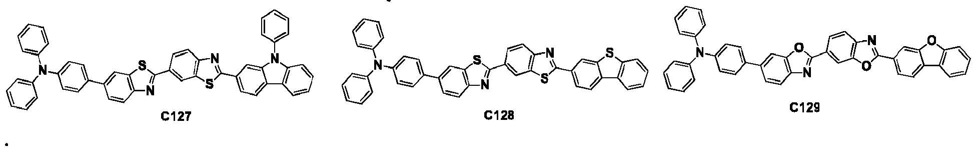

In one specific embodiment, the organic compound is a light extraction layer material.

The present disclosure further relates to a mixture that includes at least one organic compound as described above, and at least one organic functional material. The at least one organic functional material may be selected from a hole injection material (HIM), a hole transport material (HTM), an electron transport material (ETM), an electron injection material (EIM), an electron blocking material (EBM), a hole blocking material (HBM), a light-emitting material (emitter), a host material, or an organic dye. Detailed descriptions of these organic functional materials can refer to WO 2010/135519 A1, US 2009/0134784 A1, and WO 2011/110277 A1, all contents of these three patent applications are incorporated herein by reference in their entirety.

The present disclosure further relates to a composition that includes at least one organic compound or the mixture as described above, and at least one organic solvent. The at least one organic solvent is any one selected from a group consisting of an aromatic-based or a heteroaromatic-based compound, an ester-based compound, an aromatic ketone-based or an aromatic ether-based compound, an aliphatic ketone-based or an aliphatic ether-based compound, an alicyclic-based or an olefin-based compound, or a borate ester-based or a phosphate ester-based compound, or a mixture of two or more of the solvents above.

In one preferable embodiment, according to the composition of the present disclosure, at least one organic solvent is selected from an aromatic-based or a heteroaromatic-based solvent.

Examples of the aromatic-based or the heteroaromatic-based solvent suitable for the present disclosure include, but not limited to, p-diisopropylbenzene, henylpentane, 1,2,3,4-tetrahydronaphthalene, cyclohexylbenzene, 1-chloronaphthalene, 1,4-dimethylnaphthalene, 3-isopropylbiphenyl, p-methyl cumene, dipentyl benzene, tripentyl benzene, pentyltoluene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, 1,4-diisopropylbenzene, cyclohexylbenzene, benzylbutylbenzene, 2,6-dimethylnaphthalene, 3-isopropylbiphenyl, p-methyl cumene, 1-methylnaphthalene, 1,2,4-trichlorobenzene, 4,4'-difluorodiphenylmethane, methyl isoeugenol, diphenylmethane, 2-phenylpyridine, 3-phenylpyridine, N-methyldiphenylamine, 4-isopropylbiphenyl, diphenyldichloromethane, 4-(3-phenylpropyl)pyridine, benzyl benzoate, 1,1-bis(3,4-dimethylphenyl)ethane, 2-isopropylnaphthalene, quinoline, isoquinoline, methyl 2-furoate, ethyl 2-furoate, and the like.

Examples of the aromatic ketone-based solvent suitable for the present disclosure include, but not limited to, 1-tetralone, beta-tetralone, 2-(phenylepoxy)tetralone, 6-(methoxy)tetralone, acetophenone, propiophenone, benzophenone, and derivatives thereof, such as 4-methylacetophenone, 3-methylacetophenone, 2-methylacetophenone, 4-methylphenylacetone, 3-methylphenylacetone, 2-methylphenylacetone, and the like.

Examples of the aromatic ether-based solvent suitable for the present disclosure include, but not limited to, 3-phenoxytoluene, butoxybenzene, p-anisaldehyde dimethylacetal, tetrahydro-2-phenoxy-2H-pyran, 1,2-dimethoxy-4-(1 -propenyl)benzene, 1,4-benzodioxan, 1,3-dipropylbenzene, 2,5-dimethoxytoluene, 4-ethylphenetole, 1,3-dipropoxybenzene, 1,2,4-trimethoxybenzene, 4-(1-propenyl)-1,2-dimethoxybenzene, 1,3-dimethoxybenzene, glycidyl phenyl ether, dibenzyl ether, 4-tert-butylanisole, trans-p-propenylanisole, 1,2-dimethoxybenzene, 1-methoxynaphthalene, diphenyl ether, 2-phenoxymethyl ether, 2-phenoxytetrahydrofuran, and ethyl-2-naphthyl ether.

Examples of the aliphatic ketone-based solvent suitable for the present disclosure include, but not limited to, 2-nonone, 3-nonone, 5-nonone, 2-decanone, 2,5-hexanedione, 2,6,8-trimethyl-4-nonone, fenchone, phorone, isophorone, di-n-pentyl ketone, and the like. Examples of the aliphatic ether-based organic solvent suitable for the present disclosure include, but not limited to, amyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethylene glycol ethyl methyl ether, triethylene glycol butyl methyl ether, tripropylene glycol dimethyl ether, tetraethylene glycol dimethyl ether, and the like.

Examples of the ester-based solvent suitable for the present disclosure include, but not limited to, octanoate, sebacate, stearate, benzoate, phenylacetate, cinnamate, oxalate, maleate, alkyl lactone, oleate, and the like. In particular, octyl octanoate, diethyl sebacate, diallyl phthalate, and isononyl isononanoate are preferred.

The solvents can be used alone or as a mixture of two or more organic solvents.

In some preferable embodiments, the composition of the present disclosure includes at least one organic compound or the mixture as described above, and at least one organic solvent, and further includes another organic solvent. Examples of another organic solvent include, but not limited to, methanol, ethanol, 2-methoxyethanol, dichloromethane, chloroform, chlorobenzene, o-dichlorobenzene, tetrahydrofuran, anisole, morpholine, toluene, o-xylene, m-xylene, p-xylene, 1,4-dioxane, acetone, methyl ethyl ketone, 1,2-dichloroethane, 3-phenoxytoluene, 1,1,1-trichloroethane, 1,1,2,2-tetrachloroethane, ethyl acetate, butyl acetate, dimethylformamide, dimethylacetamide, dimethyl sulfoxide, tetralin, naphthane, indene, and/or mixtures thereof.

In some preferable embodiments, organic solvents particularly suitable for the present disclosure have Hansen solubility parameters in the following ranges:
δd (dispersion force) ranging from 17.0 MPa^{1/2} to 23.2 MPa^{1/2}, preferably ranging from 18.5 to 21.0 MPa^{1/2};
δp (polarity force) ranging from 0.2 MPa^{1/2} to 12.5 MPa^{1/2}, preferably ranging from 2.0 to 6.0 MPa^{1/2}; and
δh (hydrogen bonding force) ranging from 0.9 MPa^{1/2} to 14.2 MPa^{1/2}, preferably ranging from 2.0 to 6.0 MPa^{1/2}.

According to the composition of the present disclosure, when selecting organic solvents, their boiling points need to be considered. In the present disclosure, the boiling point of the organic solvents are greater than or equal to 150 °C, preferably greater than or equal to 180 °C, more preferably greater than or equal to 200 °C, further preferably greater than or equal to 250 °C, most preferably greater than or equal to 300 °C. The boiling point of the organic solvents in the above ranges is beneficial to preventing nozzles of inkjet printing heads from clogging. The organic solvents can evaporate from a solvent system to form films containing functional materials.

In one preferable embodiment, the composition according to the present disclosure is a solution.

In another preferable embodiment, the composition according to the present disclosure is a suspension.

The composition in the embodiments of the present disclosure may include from 0.01 to 10 wt% of the compound or the mixture, based on the weight of the composition, preferably 0.1 to 15 wt%, more preferably 0.2 to 5 wt%, most preferably 0.25 to 3 wt%.

The present disclosure further relates to the use of the composition as a coating material or printing ink for preparing organic electronic devices. In particular, the use in the preparation method by printing or coating is preferred.

Suitable printing or coating technologies include, but not limited to, inkjet printing, nozzle printing, letterpress printing, screen printing, dip coating, rotating coating, scraper coating, roller printing, torsion roller printing, lithographic printing, flexographic printing, rotary printing, spray coating, brush coating, transfer printing, slit extrusion coating, and the like. Gravure printing, nozzle printing, and inkjet printing are preferred. The solution or the suspension may further include one or more components, such as a surface active compound, a lubricant, a wetting agent, a dispersant, a hydrophobic agent, an adhesive, or the like, for adjusting viscosity, improving forming performance of a film and adhesiveness. Regarding printing technology and its requirements for solutions, such as solvents, concentration, viscosity, and the like.

The present disclosure further relates to a light extraction layer material, which is selected from the organic compound, the mixture, or the composition as described above.

The present disclosure further relates to the use of the above-mentioned organic compound, the mixture, and the composition in organic electroluminescent devices. Technical solutions are as follows:

An organic electroluminescent device including two electrodes, one or more organic functional layers disposed between the two electrodes, and a light-extracting layer disposed at a surface of one of the two electrodes and away from the organic functional layers. Materials of the light-extracting layer include a compound represented by formula (1): 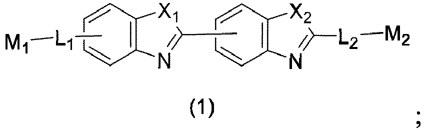 wherein,
X₁ and X₂ are independently selected from O, S, or NR₁;
L₁ and L₂ are independently selected from a single bond, a substituted or unsubstituted aromatic group having 6 to 30 ring atoms, or a substituted or unsubstituted heteroaromatic group having 5 to 30 ring atoms;
M₁ and M₂ are independently selected from a substituted or unsubstituted aromatic group having 6 to 60 ring atoms, a substituted or unsubstituted heteroaromatic group having 5 to 60 ring atoms, or a substituted or unsubstituted amino group; and
R₁ at each occurrence is independently selected from -H, -D, a linear alkyl group having 1 to 20 carbon atoms, a branched alkyl group having 3 to 20 carbon atoms, an alkenyl group having 2 to 20 carbon atoms, a cyclic alkyl group having 3 to 20 carbon atoms, a substituted or unsubstituted aromatic group having 6 to 30 ring atoms, or a substituted or unsubstituted heteroaromatic group having 5 to 30 ring atoms.

Further description about the formula (1) is the same as the description mentioned above.

In one embodiment, according to the organic electroluminescent device of the present disclosure, the light-extracting layer is disposed on a surface of a cathode away from the organic functional layers.

In some embodiments, according to the organic electroluminescent device of the present disclosure, the materials of the light-extracting layer have the refractive index greater than 1.7 at the wavelength of 630 nm, preferably greater than 1.78, more preferably greater than 1.83.

In some embodiments, according to the organic electroluminescent device of the present disclosure, the materials of the light-extracting layer need to have small extinction coefficient at the wavelength greater than 430 nm, making the light-extracting layer have higher transmissivity to visible light, thus reducing the impact on light output efficiency of devices. The extinction coefficient is less than 0.1 at the wavelength of 430 nm, preferably less than 0.003, more preferably less than 0.001.

In some preferable embodiments, according to the organic electroluminescent device of the present disclosure, the light-extracting layer has larger extinction coefficient in the wavelength range less than or equal to 400 nm. At the wavelength of 350 nm, the extinction coefficient is preferably greater than or equal to 0.3, more preferably greater than or equal to 0.5, further preferably greater than or equal to 0.7, most preferably greater than or equal to 1.0.

In some embodiments, according to the organic electroluminescent device of the present disclosure, the light-extracting layer including the organic compound has a thickness ranging from 10 nm to 200 nm, preferably 20 nm to 150 nm, more preferably 30 nm to 100 nm, most preferably 40 nm to 90 nm.

In some embodiments, according to the organic electroluminescent device of the present disclosure, the materials in the light-extracting layer need to have a higher glass transition temperature to enhance thermal stability of the materials of the light-extracting layer. In some preferable embodiments, the glass transition temperature (Tg) is greater than or equal to 100 °C, preferably greater than or equal to 120 °C, more preferably greater than or equal to 140 °C, most preferably greater than or equal to 160 °C.

In one embodiment, the organic electroluminescent device according to the present disclosure includes one or more organic functional layers selected from one or more layers consisting of an electron injection layer, an electron transport layer, a hole injection layer, a hole transport layer, and a light-emitting layer, and at least one light-emitting layer is included.

In some preferable embodiments, a light-emitting material of the light-emitting layer of the organic electroluminescent device according to the present disclosure is selected from a singlet state emitter, a triplet state emitter, or a TADF material.

In some preferable embodiments, the organic functional layers of the organic electroluminescent device according to the present disclosure are selected from a hole transport layer, a light-emitting layer, and an electron transport layer.

In some more preferable embodiments, the organic functional layers of the organic electroluminescent device according to the present disclosure are selected from a hole injection layer, a hole transport layer, a light-emitting layer, an electron transport layer, and an electron injection layer.

The singlet state emitter, the triplet state emitter, and the TADF material are described in WO 2017/092619A.

Further, the organic functional layers of the organic electroluminescent device according to the present disclosure includes a light-emitting layer. Furthermore, the material in the light-emitting layer is a triplet state light-emitting material.

In one embodiment, the triplet state light-emitting material has a structure represented by the following formula: 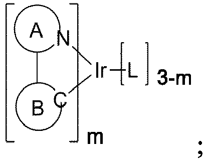
in which m is selected from 1, 2, or 3;
ring A is selected from a substituted or unsubstituted nitrogen-containing heteroaromatic group having 5 to 30 ring atoms;
ring B is selected from a substituted or unsubstituted aromatic group having 6 to 30 ring atoms or a substituted or unsubstituted heteroaromatic group having 6 to 30 ring atoms; and
L is a monovalent anionic organic ligand.

In one embodiment, the ring A is selected from a group consisting of the following groups: 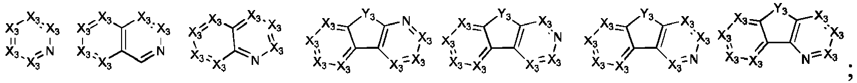 in which N indicates a linking site.

In one embodiment, the ring B is selected from a group consisting of the following groups: 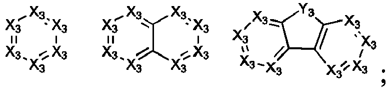 wherein,
X₃ at each occurrence is independently selected from CR₁₄ or N;
Y₃ at each occurrence is selected from CR₁₅R₁₆, NR₁₇, O, S, S=O, SO₂, PR₁₇, BR₁₇, or SiR₁₅R₁₆; and
R₁₄ to R₁₇ at each occurrence are independently selected from a hydrogen atom, a deuterium atom, a linear alkyl group having 1 to 20 carbon atoms, a branched or cyclic alkyl group having 3 to 20 carbon atoms, a cyano group, a nitro group, CF₃, OCF₃, Cl, Br, F, a substituted or unsubstituted aromatic group having 6 to 30 ring atoms, a substituted or unsubstituted heteroaromatic group having 5 to 30 ring atoms, or any combination of these groups.

Further, the triplet state light-emitting material has a structure represented by any one of the following formulae: 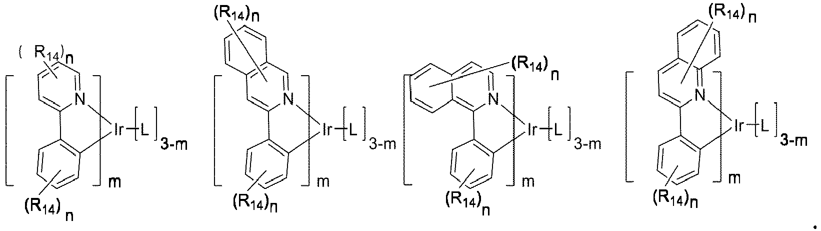 Further, R₁₄ at each occurrence is independently selected from a hydrogen atom, a deuterium atom, a linear alkyl group having 1 to 10 carbon atoms, a branched or cyclic alkyl group having 3 to 10 carbon atoms, a cyano group, a nitro group, -CF₃, -OCF₃, -Cl, -Br, -F, a substituted or unsubstituted aromatic group having 6 to 20 ring atoms, a substituted or unsubstituted heteroaromatic group having 5 to 20 ring atoms, or any combination of these groups; and
n at each occurrence is independently an integer from 0 to 5.

The triplet state light-emitting material according to the present disclosure has a structure selected from a group consisting of the following structures:

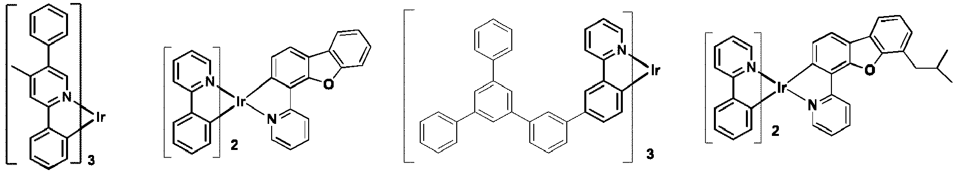

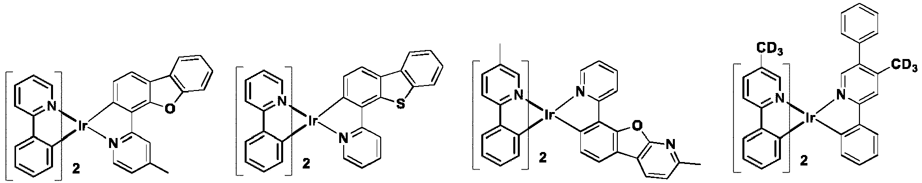

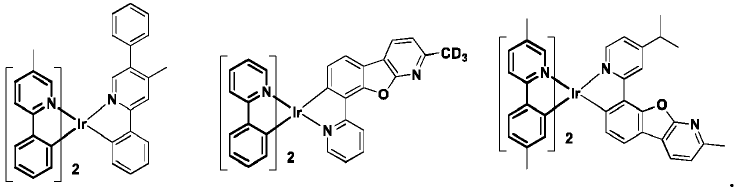

The organic electronic device according to the present disclosure may be selected from, but not limited to, an organic light-emitting diode (OLED), an organic photovoltaic cell, an organic light-emitting cell, an organic field-effect transistor, an organic light-emitting field-effect transistor, an organic laser, an organic spintronic device, an organic sensor, an organic plasmon emitting diode, or the like. In particular, the OLED is preferred.

The following will provide a description of the cathode and the anode in the organic light-emitting diode.

The anode may include a conductive metal, a metal oxide, or a conductive polymer. Holes in the anode can be easily injected into a hole injection layer (HIL), a hole transport layer (HTL), or a light-emitting layer. In one embodiment, an absolute value of a difference between work function of the anode and HOMO energy level or valence band energy level of an emitter of the light-emitting layer, or a p-type semiconductor of the HIL, the HTL, or an electron blocking layer (EBL) is less than 0.5 eV, preferably less than 0.3 eV, more preferably less than 0.2 eV. Examples of materials of the anode include, but not limited to, Al, Cu, Au, Ag, Mg, Fe, Co, Ni, Mn, Pd, Pt, ITO, zinc oxide doped with aluminum (AZO), and the like. Other suitable anode materials known in the art can be easily chose and used by those skilled in the art. The materials of the anode can be deposited using any suitable technology, such as a suitable physical vapor deposition method including RF magnetron sputtering, vacuum thermal evaporation, electron beam (e-beam), or the like. In some embodiments, the anode is a patterned structure. A patterned ITO conductive substrate can be commercially available and used to prepare the devices of the present disclosure.

The cathode may include a conductive metal or a metal oxide. Electrons in the cathode can be easily injected into an electron injection layer (EIL), an electron transport layer (ETL), or the light-emitting layer. In one embodiment, an absolute value of a difference between work function of the cathode and LUMO energy level or valence band energy level of an emitter of the light-emitting layer, or a n-type semiconductor material of the EIL, the ETL, or a hole blocking layer (HBL) is less than 0.5 eV, preferably less than 0.3 eV, more preferably less than 0.2 eV. In principle, all materials that can be used for the cathode of OLEDs can be used as materials of the cathode of the devices according to the present disclosure. Examples of the materials of the cathode include, but not limited to, Al, Au, Ag, Ca, Ba, Mg, LiF/Al, MgAg alloy, BaF₂/Al, Cu, Fe, Co, Ni, Mn, Pd, Pt, ITO, and the like. The materials of the cathode can be deposited using any suitable technology, such as a suitable physical vapor deposition method including RF magnetron sputtering, vacuum thermal evaporation, electron beam (e-beam), or the like.

The light extraction layer material needs to have a suitable energy level structure, so that the light-extracting layer has stronger absorption to light at regions with wavelengths less than 400 nm, and has weaker or near zero absorption to visible light with wavelengths greater than 400 nm, so as to avoid damage to materials in the devices caused by high-energy light irradiation in subsequent processes. Moreover, the light extraction layer material has higher refractive index, which can effectively export visible light emission and improve luminescence efficiency of organic electronic light-emitting devices. When the reflectivity at the interface between the light extraction layer material and adjacent electrodes is larger, light interference has significant influence. Therefore, the refractive index of the light extraction layer material is preferably greater than that of the adjacent electrodes.

In one embodiment, the light-extracting layer of the organic electroluminescent device according to the present disclosure, particularly the light-extracting layer of the organic light-emitting diode is disposed on a surface of the cathode.

In some preferable embodiments, the light-extracting layer including the organic compound of the organic electroluminescent device according to the present disclosure has a thickness ranging from 10 nm to 200 nm, preferably 20 nm to 150 nm, more preferably 30 nm to 100 nm, most preferably 40 nm to 90 nm.

The present disclosure further relates to the use of the electroluminescent device according to the present disclosure in various electronic apparatuses, which include, but not limited to, display devices, illumination devices, light sources, sensors, and the like.

The following will provide a description of the present disclosure in conjunction with preferable examples, but not limited to the following examples. It should be understood that the attached claims summarize the scope of the present disclosure. Under the guidance of the concept of the present disclosure, those skilled in the art should be aware that certain changes made to each example of the present disclosure will be covered by the spirit and scope of the claims of the present disclosure.

### Examples

The following will provide a further detailed description of the organic compounds of the present disclosure and methods for preparing the same in conjunction with specific examples. Raw materials used in the following examples are all commercially available products, unless otherwise specified.

### Example 1

This example provides an organic compound C26 with a specific synthesis route as follows:

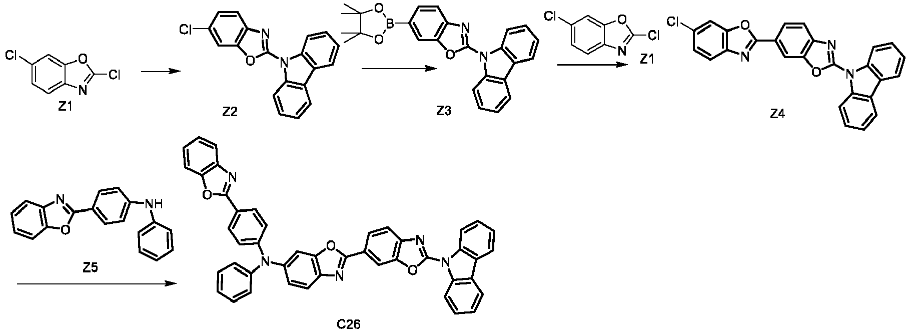

Carbazole (3.34 g, 20 mmol) was dissolved in anhydrous DMF, cooled to 0 °C, then sodium hydride was added. After the reaction solution was continuously stirred for half an hour, intermediate Z1 (3.74 g, 20 mmol) was added, then naturally raise the temperature of the reaction solution and track it by TLC. After the reaction was completed, deionized water was added to the reaction solution, then the reaction solution was extracted with ethyl acetate to obtain an organic layer. The organic layer was washed three times with water, concentrated, and purified by silica gel column chromatography to obtain 5.4 g of intermediate Z2 with yield of 85%.

The intermediate Z2 (4.77 g, 15 mmol) was dissolved in anhydrous tetrahydrofuran, then potassium acetate (3 g, 30 mmol), bis(pinacolato)diboron (4.06 g, 16 mmol), and bis(triphenylphosphine)palladium(II) chloride (0.32 g, 0.45 mmol) were added thereto. The reaction atmosphere was replaced with nitrogen, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 4.8 g of intermediate Z3 with yield of 78%.

Z1 (1.87 g, 10 mmol) and Z3 (4.1 g, 10 mmol) were dissolved in anhydrous toluene and methanol, then potassium carbonate (2.76 g, 20 mmol) and tetrakis(triphenylphosphine)palladium (0.35 g, 0.3 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 3.13 g of product Z4 with yield of 72%.

Compounds Z4 (2.61 g, 6 mmol) and Z5 (1.72 g, 6 mmol) were dissolved in anhydrous toluene, then sodium tert-butoxide (0.67 g, 7 mmol) and tris(dibenzylideneacetone)dipalladium (0.16 g, 0.18 mmol) were added thereto. After the reaction atmosphere was replaced with nitrogen three times, tri-tert-butylphosphine (0.18 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 3.33 g of product C26 with yield of 81%. MS: 685 [M⁺].

### Example 2

This example provides an organic compound C1 with a specific synthesis route as follows:

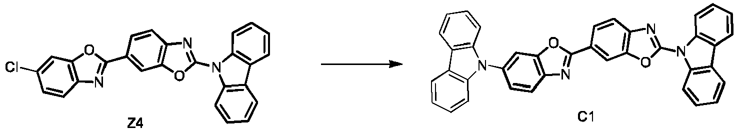

Compound Z4 (4.35 g, 10 mmol) and carbazole (1.84 g, 11 mmol) were dissolved in anhydrous toluene, then sodium tert-butoxide (1.15 g, 12 mmol) and tris(dibenzylideneacetone)dipalladium (0.27 g, 0.3 mmol) were added thereto. After the reaction atmosphere was replaced with nitrogen three times, tri-tert-butylphosphine (0.3 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 3.9 g of product C1 with yield of 69%. MS: 566 [M⁺].

### Example 3

This example provides an organic compound C3 with a specific synthesis route as follows:

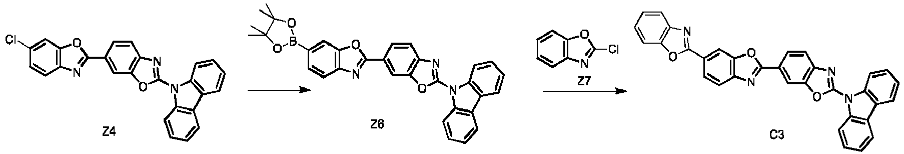

Intermediate Z4 (4.35 g, 10 mmol) was dissolved in anhydrous tetrahydrofuran, potassium acetate (1.96 g, 20 mmol), bis(pinacolato)diboron (3.05 g, 12 mmol), and bis(triphenylphosphine)palladium(II) chloride (0.27 g, 0.3 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 3.48 g of intermediate Z6 with yield of 66%.

Z6 (3.16 g, 6 mmol) and Z7 (0.92 g, 6 mmol) were dissolved in anhydrous toluene and methanol, then potassium carbonate (1.66 g, 12 mmol) and tetrakis(triphenylphosphine)palladium (0.2 g, 0.18 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 2.27 g of product C3 with yield of 73%. MS: 518 [M⁺].

### Example 4

This example provides an organic compound C7 with a specific synthesis route as follows:

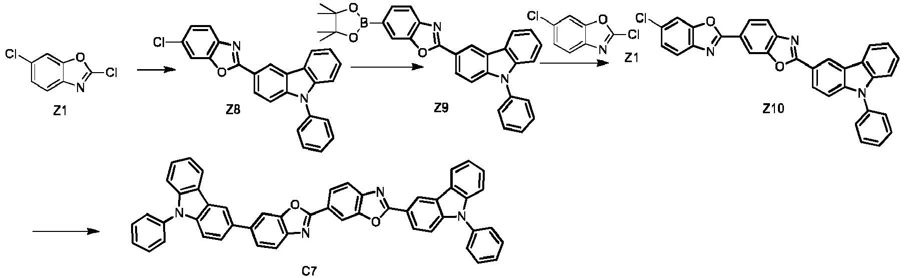

Z1 (5.61 g, 30 mmol) and 9-phenyl-9H-carbazol-3-ylboronic acid (8.61 g, 30 mmol) were dissolved in anhydrous toluene and methanol, then potassium carbonate (12.42 g, 90 mmol) and tetrakis(triphenylphosphine)palladium (1.04 g, 0.9 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 9.1 g of product Z8 with yield of 77%.

Intermediate Z8 (7.88 g, 20 mmol) was dissolved in anhydrous tetrahydrofuran, potassium acetate (3.92 g, 40 mmol), bis(pinacolato)diboron (5.6 g, 22 mmol), and bis(triphenylphosphine)palladium(II) chloride (0.42 g, 0.6 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 8.16 g of intermediate Z9 with yield of 84%.

Z9 (7.29 g, 15 mmol) and Z1 (2.8 g, 15 mmol) were dissolved in anhydrous toluene and methanol, then potassium carbonate (4.14 g, 30 mmol) and tetrakis(triphenylphosphine)palladium (0.52 g, 0.45 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 4.98 g of product Z10 with yield of 65%.

Z10 (4.1 g, 8 mmol) and 9-phenyl-9H-carbazol-3-ylboronic acid (2.3 g, 8 mmol) were dissolved in anhydrous toluene and methanol, then potassium carbonate (2.21 g, 16 mmol) and tetrakis(triphenylphosphine)palladium (0.28 g, 0.24 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 3.5 g of product C7 with yield of 61%. MS: 718 [M⁺].

### Example 5

This example provides an organic compound C101 with a specific synthesis route as follows:

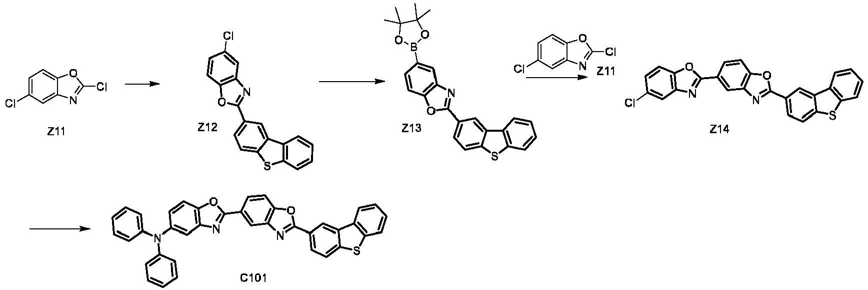

Z11 (5.61 g, 30 mmol) and dibenzothiopheneboronic acid (6.84 g, 30 mmol) were dissolved in anhydrous toluene and methanol, then (8.28 g, 60 mmol) and tetrakis(triphenylphosphine)palladium (1.04 g, 0.9 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 8.04 g of product Z12 with yield of 80%.

Intermediate Z12 (6.7 g, 20 mmol) was dissolved in anhydrous tetrahydrofuran, potassium acetate (3.92 g, 40 mmol), bis(pinacolato)diboron (5.6 g, 22 mmol), and bis(triphenylphosphine)palladium(II) chloride (0.42 g, 0.6 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 5.29 g of intermediate Z13 with yield of 62%.

Z13 (4.27 g, 10 mmol) and Z11 (1.87 g, 10 mmol) were dissolved in anhydrous toluene and methanol, then potassium carbonate (2.76 g, 20 mmol) and tetrakis(triphenylphosphine)palladium (0.35 g, 0.3 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 3.25 g of product Z14 with yield of 72%.

Compound Z14 (3.16 g, 7 mmol) and diphenylamine (1.18 g, 7 mmol) were dissolved in anhydrous toluene, then sodium tert-butoxide (0.77 g, 8 mmol) and tris(dibenzylideneacetone)dipalladium (0.19 g, 0.21 mmol) were added thereto. After the reaction atmosphere was replaced with nitrogen three times, tri-tert-butylphosphine (0.21 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 3.56 g of product C101 with yield of 87%. MS: 585 [M+].

### Example 6

This example provides an organic compound C49 with a specific synthesis route as follows:

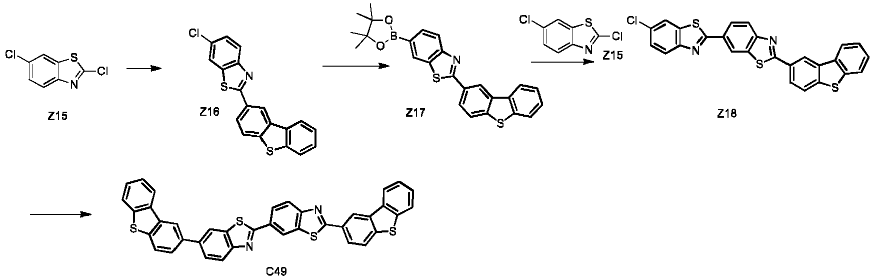

Z15 (4.04 g, 20 mmol) and dibenzothiopheneboronic acid (4.56 g, 20 mmol) were dissolved in anhydrous toluene and methanol, then potassium carbonate (5.52 g, 40 mmol) and tetrakis(triphenylphosphine)palladium (0.69 g, 0.6 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 5.46 g of product Z16 with yield of 78%.

Intermediate Z16 (4.9 g, 14 mmol) was dissolved in anhydrous tetrahydrofuran, potassium acetate (2.75 g, 28 mmol), bis(pinacolato)diboron (4.06 g, 16 mmol), and bis(triphenylphosphine)palladium(II) chloride (0.29 g, 0.42 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 5.15 g of intermediate Z17 with yield of 83%.

Z17 (4.43 g, 10 mmol) and Z15 (2.03 g, 10 mmol) were dissolved in anhydrous toluene and methanol, then potassium carbonate (2.76 g, 20 mmol) and tetrakis(triphenylphosphine)palladium (0.35 g, 0.3 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 3.63 g of product Z18 with yield of 75%.

Z18 (3.39 g, 7 mmol) and dibenzothiopheneboronic acid (1.6 g, 7 mmol) were dissolved in anhydrous toluene and methanol, then potassium carbonate (1.93 g, 14 mmol) and tetrakis(triphenylphosphine)palladium (0.24 g, 0.21 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 2.74 g of product C49 with yield of 62%. MS: 632 [M⁺].

### Example 7

This example provides an organic compound C50 with a specific synthesis route as follows:

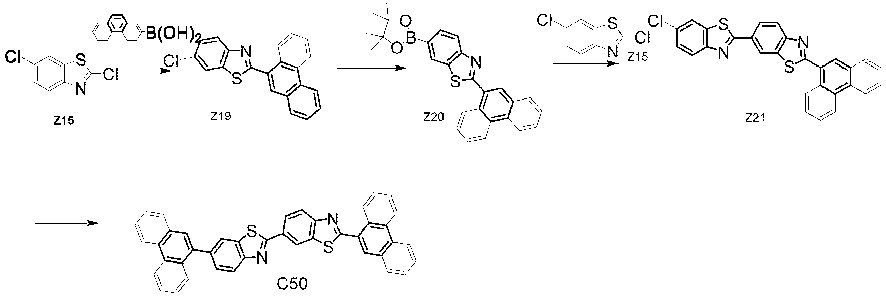

Z15 (5.07 g, 25 mmol) and 9-phenanthrenylboronic acid (5.55 g, 25 mmol) were dissolved in anhydrous toluene and methanol, then potassium carbonate (6.9 g, 50 mmol) and tetrakis(triphenylphosphine)palladium (0.87 g, 0.75 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 6.21 g of product Z19 with yield of 72%.

Intermediate Z19 (5.86 g, 17 mmol) was dissolved in anhydrous tetrahydrofuran, potassium acetate (3.33 g, 34 mmol), bis(pinacolato)diboron (4.83 g, 19 mmol), and bis(triphenylphosphine)palladium(II) chloride (0.36 g, 0.51 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 4.53 g of intermediate Z20 with yield of 61%.

Z20 (4.37 g, 10 mmol) and Z15 (2.03 g, 10 mmol) were dissolved in anhydrous toluene and methanol, then potassium carbonate (2.76 g, 20 mmol) and tetrakis(triphenylphosphine)palladium (0.35 g, 0.3 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 3.06 g of product Z21 with yield of 64%.

Z21 (2.87 g, 6 mmol) and 9-phenanthrenylboronic acid (1.33 g, 6 mmol) were dissolved in anhydrous toluene and methanol, then potassium carbonate (1.66 g, 12 mmol) and tetrakis(triphenylphosphine)palladium (0.21 g, 0.18 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 2.16 g of product C50 with yield of 58%. MS: 620 [M⁺].

### Example 8

This example provides an organic compound C56 with a specific synthesis route as follows:

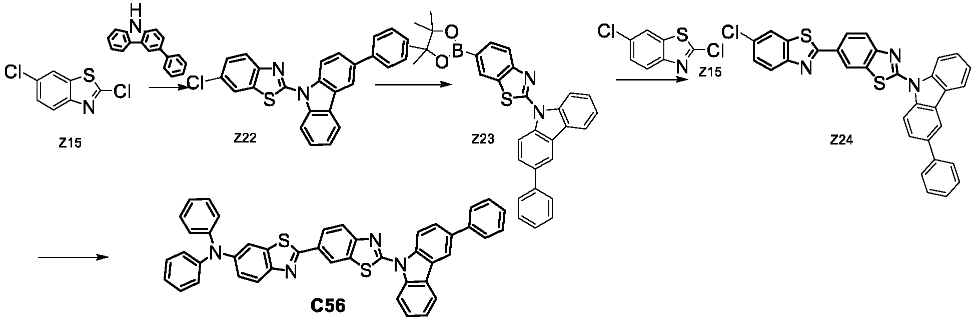

Compound Z15 (5.07 g, 25 mmol) and carbazole (6.08 g, 25 mmol) were dissolved in anhydrous toluene, then sodium tert-butoxide (2.88 g, 30 mmol) and tris(dibenzylideneacetone)dipalladium (0.69 g, 0.75 mmol) were added thereto. After the reaction atmosphere was replaced with nitrogen three times, tri-tert-butylphosphine (0.75 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 8.4 g of product Z22 with yield of 82%.

Intermediate Z22 (8.2 g, 20 mmol) was dissolved in anhydrous tetrahydrofuran, potassium acetate (3.92 g, 40 mmol), bis(pinacolato)diboron (5.6 g, 22 mmol), and bis(triphenylphosphine)palladium(II) chloride (0.42 g, 0.6 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 5.82 g of intermediate Z23 with yield of 58%.

Z23 (5.02 g, 10 mmol) and Z15 (2.03 g, 10 mmol) were dissolved in anhydrous toluene and methanol, then potassium carbonate (2.76 g, 20 mmol) and tetrakis(triphenylphosphine)palladium (0.35 g, 0.3 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 3.31 g of product Z24 with yield of 61%.

Compound Z24 (3.26 g, 6 mmol) and diphenylamine (1.01 g, 6 mmol) were dissolved in anhydrous toluene, then sodium tert-butoxide (0.67 g, 7 mmol) and tris(dibenzylideneacetone)dipalladium (0.16 g, 0.18 mmol) were added thereto. After the reaction atmosphere was replaced with nitrogen three times, tri-tert-butylphosphine (0.18 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 2.11 g of product C56 with yield of 52%. MS: 676 [M+].

### Example 9

This example provides an organic compound C82 with a specific synthesis route as follows:

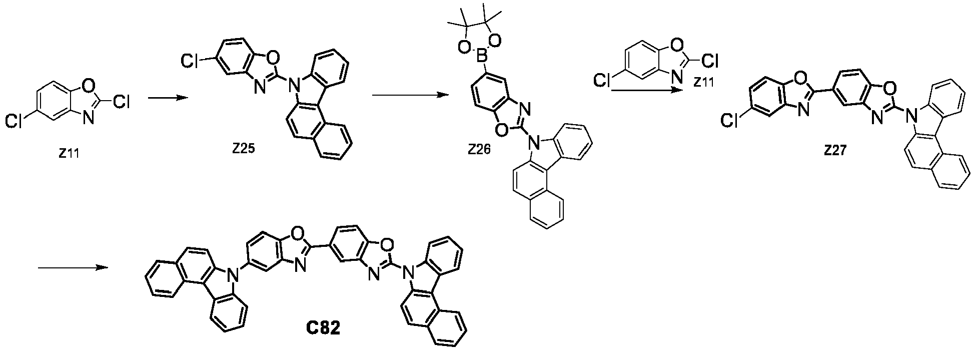

Compound Z11 (5.61 g, 30 mmol) and carbazole (6.51 g, 30 mmol) were dissolved in anhydrous toluene, then sodium tert-butoxide (3.46 g, 36 mmol) and tris(dibenzylideneacetone)dipalladium (0.82 g, 0.9 mmol) were added thereto. After the reaction atmosphere was replaced with nitrogen three times, tri-tert-butylphosphine (0.9 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 8.28 g of product Z25 with yield of 75%.

Intermediate Z25 (7.36 g, 20 mmol) was dissolved in anhydrous tetrahydrofuran, potassium acetate (3.92 g, 40 mmol), bis(pinacolato)diboron (5.6 g, 22 mmol), and bis(triphenylphosphine)palladium(II) chloride (0.42 g, 0.6 mmol) were added thereto, then raise the temperature to reflux. The reaction solution was stirred and reacted for 12 hours. After the reaction system was cooled, water was added to separate out an organic phase. The organic phase was extracted three times with ethyl acetate, concentrated under reduced pressure, and purified by silica gel column chromatography to obtain 4.97 g of intermediate Z26 with yield of 54%.

Z26 (4.6 g, 10 mmol) and Z11 (1.87 g, 10 mmol) were dissolved in anhydrous toluene and methanol, then potassium carbonate (2.76 g, 20 mmol) and tetrakis(triphenylphosphine)palladium (0.35 g, 0.3 mmol) were added thereto. The reaction atmosphere was replaced with nitrogen three times, then gradually raise the temperature to 80 °C. The reaction solution was stirred and reacted. The heat source was removed when the reactants on the TLC plate disappeared. After the reaction system was cooled, water was added to separate out an organic layer. The organic layer was extracted three times with ethyl acetate, concentrated under reduced pressure, and purified by silica gel column chromatography to obtain 2.81 g of product Z27 with yield of 58%.

Compound Z27 (2.42 g, 5 mmol) and carbazole (1.09 g, 5 mmol) were dissolved in anhydrous toluene, then sodium tert-butoxide (0.67 g, 7 mmol) and tris(dibenzylideneacetone)dipalladium (0.16 g, 0.18 mmol) were added thereto. After the reaction atmosphere was replaced with nitrogen three times, tri-tert-butylphosphine (0.18 mmol) was added, then gradually raise the temperature to 80 °C. The reaction solution was stirred and reacted for 12 hours, then the heat source was removed. After the reaction system was cooled, deionized water was added to separate out an organic layer. The organic layer was extracted three times with ethyl acetate, concentrated under reduced pressure, and purified by silica gel column chromatography to obtain 1.53 g of product C82 with yield of 46%. MS: 666 [M⁺].

### Measurement of Extinction Coefficient and Refractive Index

The compounds were respectively deposited on single crystal silicon by vacuum deposition to form 50 nm of films. The single crystal silicon was placed on an ellipsometer (ES-01) sample stage with an incidence angle of 70°. The measurement was carried out under atmospheric environment. The extinction coefficient (k) and the refractive index (n) of the compounds were obtain through fitting using the ellipsometer. The results are shown in Table 1.

**Table 1**

| Compound | Extinction coefficient @430 nm | Refractive index @630 nm |
|---|---|---|
| C26 | 0.08 | 1.90 |
| C1 | 0 | 1.85 |
| C3 | 0 | 1.88 |
| C7 | 0 | 1.88 |
| C101 | 0.02 | 1.89 |
| C49 | 0 | 1.86 |
| C50 | 0 | 1.82 |
| C56 | 0.05 | 1.90 |
| C82 | 0 | 1.87 |
| CBP | 0 | 1.74 |

The compounds of the present disclosure have weaker absorption to the light in the visible spectrum and higher absorption to the light in the ultraviolet spectrum, which can resist the damage of external light with high-energy to the components in the devices. Moreover, the higher refractive index of the compounds can ensure better light-extracting effects.

### Preparation and Characterization of OLED devices

The following will provide a detailed description of the processes for preparing the OLED devices in conjunction with specific examples. The structure of the OLED devices is as follows: ITO/Ag/ITO (anode)/HATCN/SFNFB/m-CP: Ir(p-ppy)₃/NaTzF₂/LiF/Mg: Ag/a light-extracting layer, as shown in FIG. 1. Specific preparation steps are as follows:

Cleaning a ITO conductive glass used as an anode layer, followed by ultrasonic cleaning with deionized water, acetone, and isopropanol for 15 minutes, and then treating in a plasma cleaner for 5 minutes to improve work function of electrodes. Depositing a hole injection material (HATCN) on the ITO anode layer by vacuum evaporation with an evaporation rate of 1 Å/s to obtain a hole injection layer with a thickness of 5 nm. Depositing a hole transport material (SFNFB) on the hole injection layer by vacuum evaporation to obtain a hole transport layer with a thickness of 80 nm. Depositing m-CP used as a host material and Ir(p-ppy)₃ used as a doping material on the hole transport layer by vacuum evaporation to obtain a light-emitting layer with a thickness of 30 nm, a ratio of Ir(p-ppy)₃ to m-CP is 1:9 by mass. Depositing an electron transport material (NaTzF₂) on the light-emitting layer by vacuum evaporation to obtain an electron transport layer with a thickness of 30 nm. Depositing an electron injection material (LiF) on the electron transport layer by vacuum evaporation to obtain an electron injection layer with a thickness of 1 nm. Depositing Mg doped with Ag in a ratio of 9:1 by mass on the electron injection layer by vacuum evaporation to obtain a cathode with a thickness of 15 nm. Depositing compound C26 on the cathode by vacuum evaporation to obtain a light-extracting layer with a thickness of 60 nm.
Device Example 2: C1 was substituted for compound C26 in the preparation step of the light-extracting layer in the organic electroluminescent device.
Device Example 3: C3 was substituted for compound C26 in the preparation step of the light-extracting layer in the organic electroluminescent device.
Device Example 4: C7 was substituted for compound C26 in the preparation step of the light-extracting layer in the organic electroluminescent device.
Device Example 5: C101 was substituted for compound C26 in the preparation step of the light-extracting layer in the organic electroluminescent device.
Device Example 6: C49 was substituted for compound C26 in the preparation step of the light-extracting layer in the organic electroluminescent device.
Device Example 7: C50 was substituted for compound C26 in the preparation step of the light-extracting layer in the organic electroluminescent device.
Device Example 8: C56 was substituted for compound C26 in the preparation step of the light-extracting layer in the organic electroluminescent device.
Device Example 9: C82 was substituted for compound C26 in the preparation step of the light-extracting layer in the organic electroluminescent device.
Device Comparative Example 1: CBP was substituted for compound C26 in the preparation step of the light-extracting layer in the organic electroluminescent device.
Device Comparative Example 2: Ref.-1 was substituted for compound C26 in the preparation step of the light-extracting layer in the organic electroluminescent device.

Structures of the compounds in the devices are as follows:

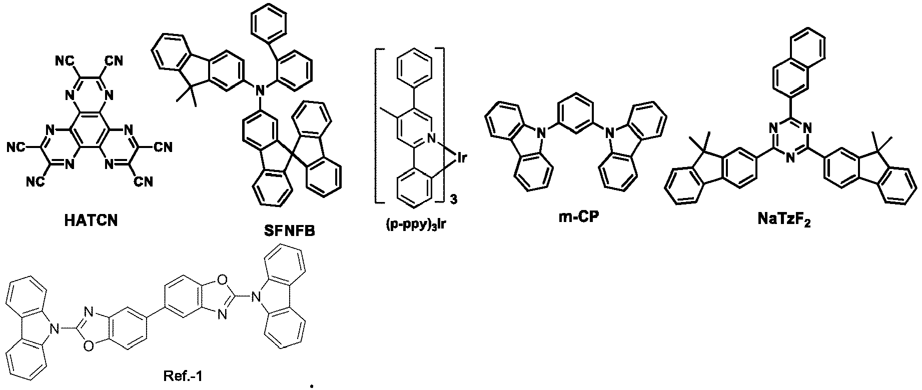

**Table 2**

| Serial number | Compound of the light-extracting layer | Luminescence efficiency |
|---|---|---|
| Device Example 1 | C26 | 1.19 |
| Device Example 2 | C1 | 1.12 |
| Device Example 3 | C3 | 1.14 |
| Device Example 4 | C7 | 1.14 |
| Device Example 5 | C101 | 1.16 |
| Device Example 6 | C49 | 1.13 |
| Device Example 7 | C50 | 1.07 |
| Device Example 8 | C56 | 1.18 |
| Device Example 9 | C82 | 1.13 |
| Comparative Example 1 | CBP | 1 |
| Comparative Example 2 | Ref.-1 | 1.03 |

The luminescence efficiency in Table 2 refers to the relative value obtained at 10 mA/cm² of current density. It can be seen from Table 2 that, compared with the compounds in comparative examples, the compounds of the present disclosure used in light-extracting layers can effectively improve luminescence efficiency of organic electroluminescent devices.

The above examples only illustrate several implementation methods of the present disclosure, which facilitates specific and detailed understanding of the technical solutions of the present disclosure, but cannot be understood as a limitation on the protection scope of the present disclosure. It should be pointed out that, for ordinary skilled in the art, several modifications and improvements can be made without departing from the concepts of the present disclosure, all of which fall within the protection scope of the present disclosure. Understandably, the technical solutions obtained by those skilled in the art through logical analysis, deduction, or limited experiments based on the technical solutions provided in the present disclosure are all within the protection scope of the claims attached to the present disclosure. Therefore, the protection scope of the present disclosure should be based on the contents of the attached claims, and the specification can be used to explain the contents of the claims.

## Claims

1. An organic compound having a structure represented by formula (1): 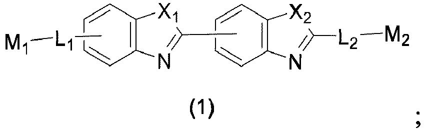 wherein,
X₁ and X₂ are independently selected from O, S, or NR₁;
L₁ and L₂ are independently selected from a single bond, a substituted or unsubstituted aromatic group having 6 to 30 ring atoms, or a substituted or unsubstituted heteroaromatic group having 5 to 30 ring atoms;
M₁ and M₂ are independently selected from a substituted or unsubstituted aromatic group having 6 to 60 ring atoms, a substituted or unsubstituted heteroaromatic group having 5 to 60 ring atoms, or a substituted or unsubstituted amino group; and
R₁ at each occurrence is independently selected from -H, -D, a linear alkyl group having 1 to 20 carbon atoms, a branched alkyl group having 3 to 20 carbon atoms, an alkenyl group having 2 to 20 carbon atoms, a cyclic alkyl group having 3 to 20 carbon atoms, a substituted or unsubstituted aromatic group having 6 to 30 ring atoms, or a substituted or unsubstituted heteroaromatic group having 5 to 30 ring atoms.

2. The organic compound of claim 1, wherein the organic compound has a structure represented by formula (2-1) or formula (2-2):

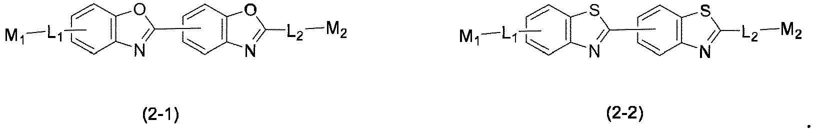

3. The organic compound of claim 1, wherein 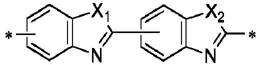 is selected from a group consisting of following structures: 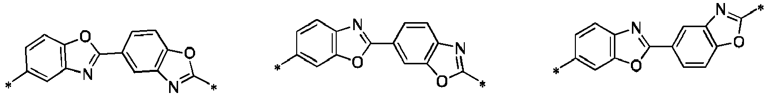 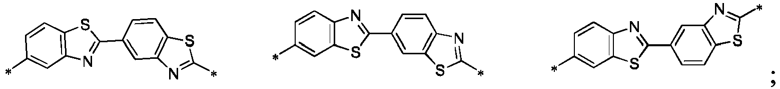 wherein * indicates a linking site.

4. The organic compound of claim 1, wherein L₁ and L₂ are independently selected from a single bond, a substituted or unsubstituted aromatic group having 6 to 20 ring atoms, or a substituted or unsubstituted heteroaromatic group having 5 to 20 ring atoms.

5. The organic compound of claim 1, wherein L₁ and L₂ are independently selected from a single bond and a group consisting of following groups: 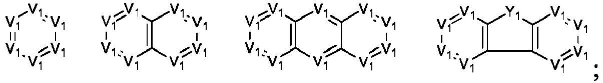 wherein,
V₁ at each occurrence is independently selected from CR₂ or N;
Y₁ is selected from NR₃, CR₄R₅, SiR₄R₅, O, S, S=O, or SO₂; and
R₂, R₃, R₄, and R₅ at each occurrence are independently selected from -H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 5 to 20 ring atoms, a substituted or unsubstituted aryloxy group having 5 to 20 ring atoms, a substituted or unsubstituted heteroaryloxy group having 5 to 20 ring atoms, or any combination of these groups.

6. The organic compound of claim 1, wherein M₁ and M₂ are independently selected from a group consisting of following groups: 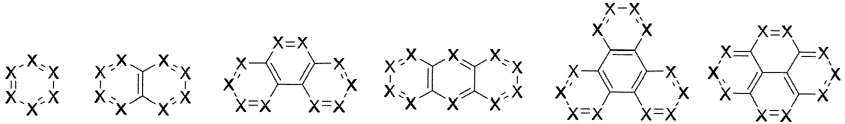 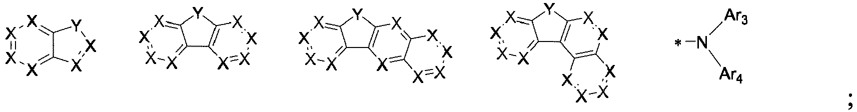 wherein,
X at each occurrence is independently selected from CR₆ or N;
Y at each occurrence is selected from NR₇, CR₈R₉, SiR₈R₉, O, S, S=O, or SO₂; and
R₆, R₇, R₈, and R₉ at each occurrence are independently selected from-H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 30 ring atoms, a substituted or unsubstituted heteroaromatic group having 5 to 30 ring atoms, a substituted or unsubstituted aryloxy group having 5 to 30 ring atoms, a substituted or unsubstituted heteroaryloxy group having 5 to 30 ring atoms, or any combination of these groups; and R₈ and R₉ form a ring or do not form a ring together;
Ar₃ and Ar₄ are independently selected from a substituted or unsubstituted aromatic group having 6 to 30 ring atoms or a substituted or unsubstituted heteroaromatic group having 5 to 30 ring atoms; and
* indicates a linking site.

7. The organic compound of claim 6, wherein Ar₃ and Ar₄ are independently selected from a group consisting of following groups: 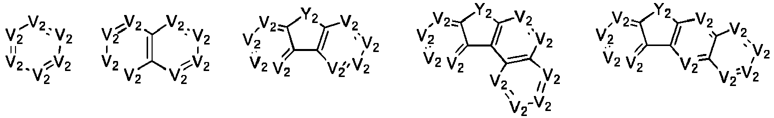 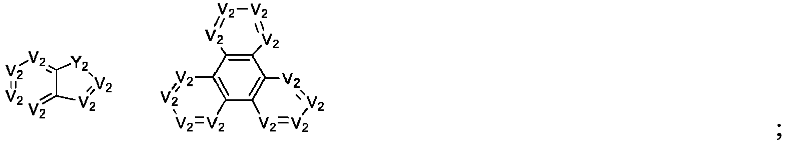 wherein,
V₂ at each occurrence is independently selected from CR₁₀ or N;
Y₂ is selected from NR₁₁, CR₁₂R₁₃, SiR₁₂R₁₃, O, S, S=O, or SO₂; and
R₁₀, R₁₁, R₁₂, and R₁₃ at each occurrence are independently selected from-H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 5 to 20 ring atoms, a substituted or unsubstituted aryloxy group having 5 to 20 ring atoms, a substituted or unsubstituted heteroaryloxy group having 5 to 20 ring atoms, or any combination of these groups; and R₁₂ and R₁₃ form a ring or do not form a ring together.

8. The organic compound of claim 1, wherein M₁ and M₂ are independently selected from a group consisting of following groups: 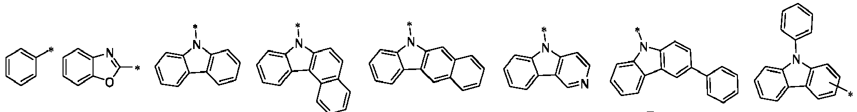 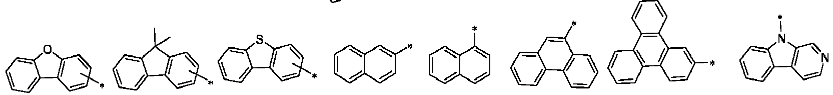 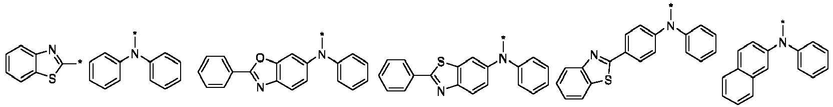 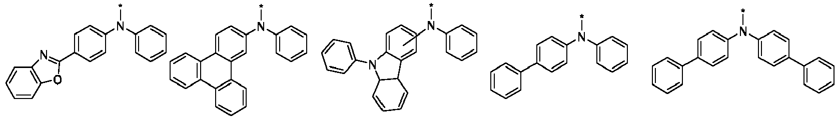 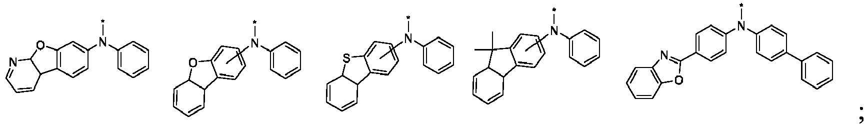 and
* indicates a linking site.

9. The organic compound of claim 6, wherein the organic compound has a structure represented by any one of formulae (3-1) to (3-7):

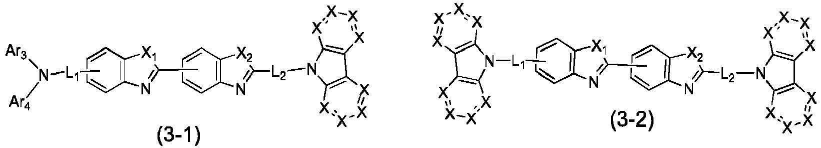

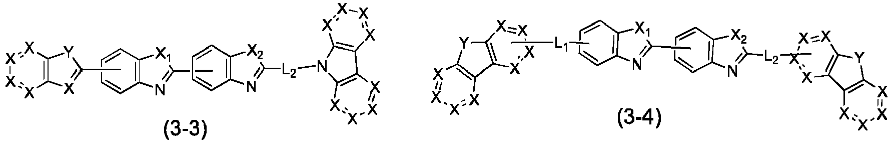

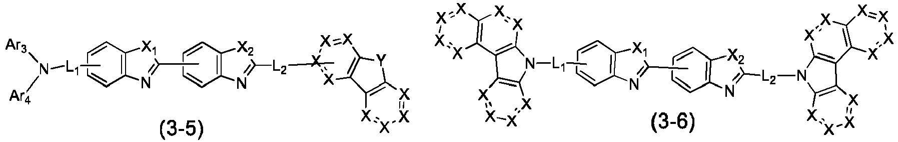

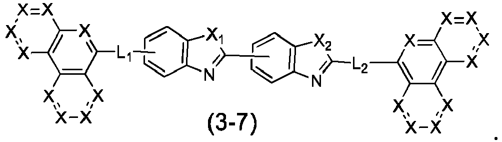

10. The organic compound of claim 1, wherein the organic compound has a structure selected from a group consisting of following structures:

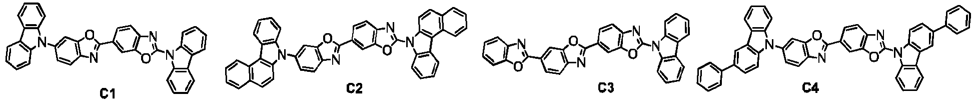

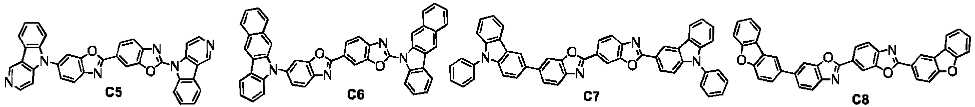

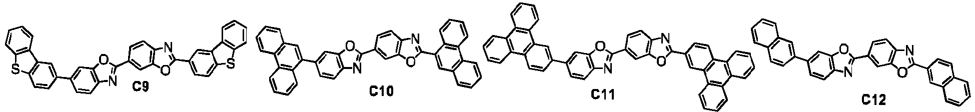

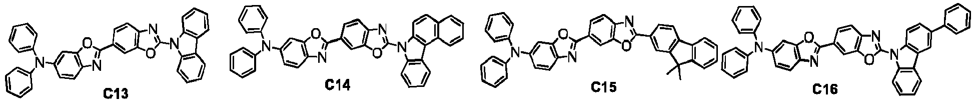

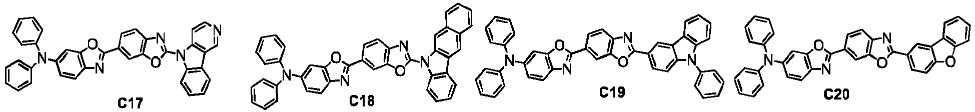

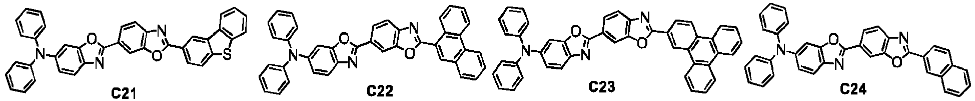

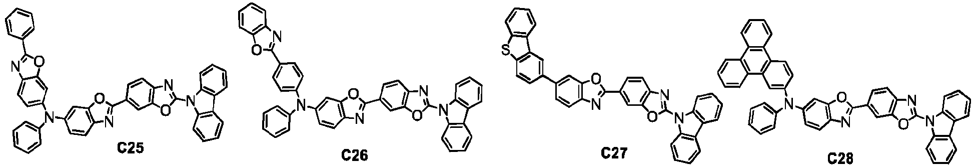

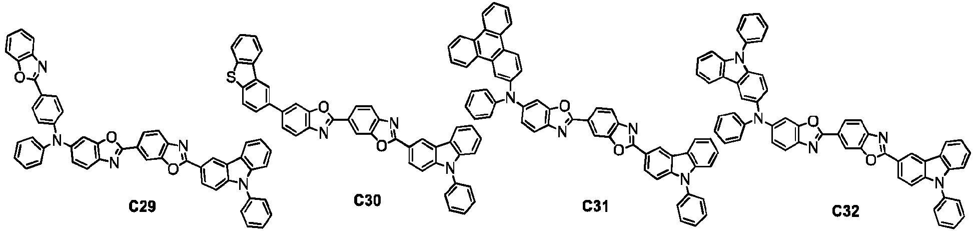

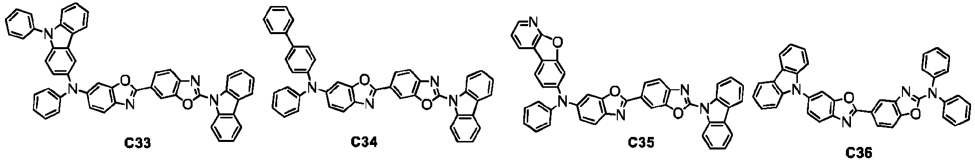

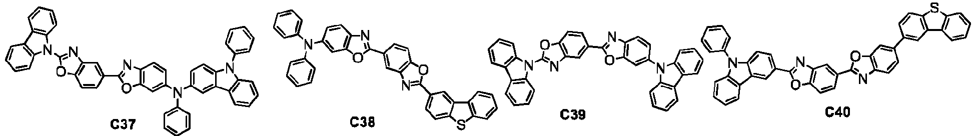

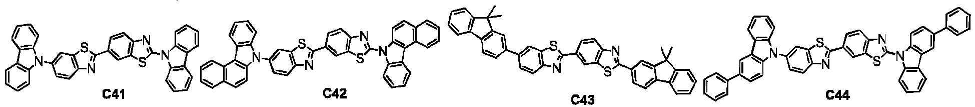

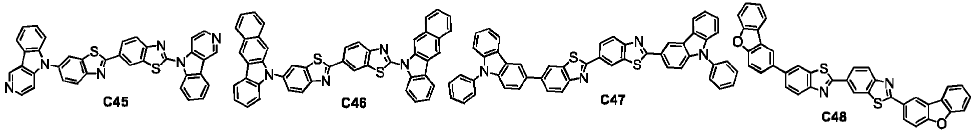

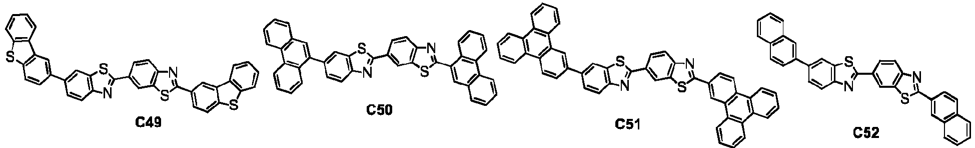

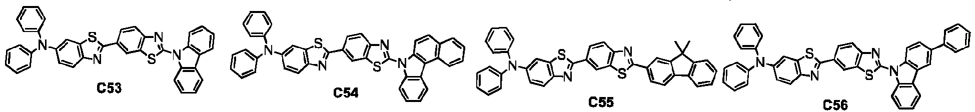

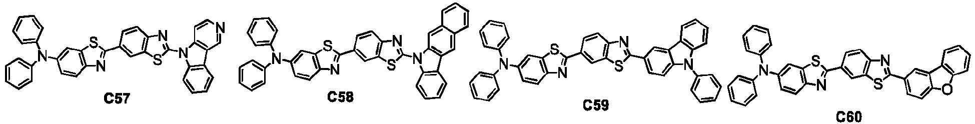

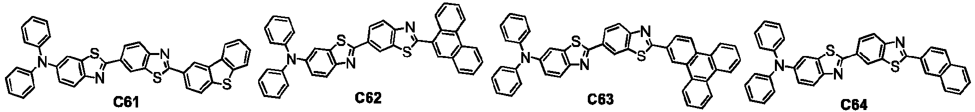

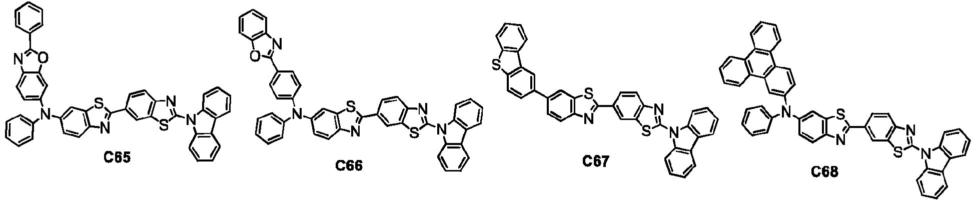

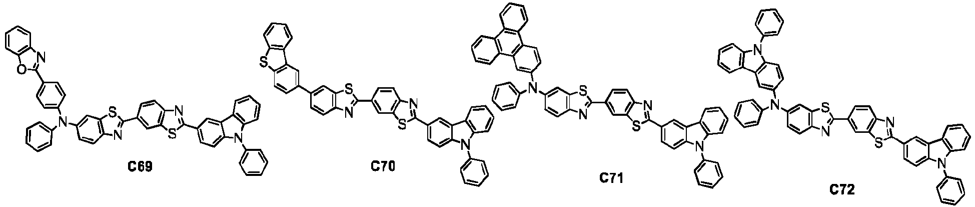

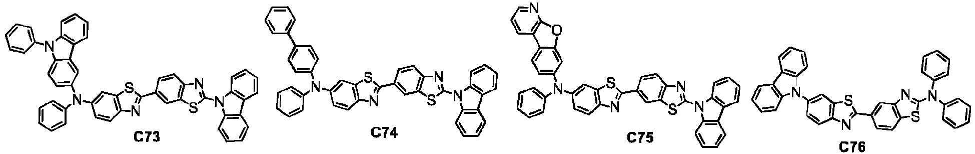

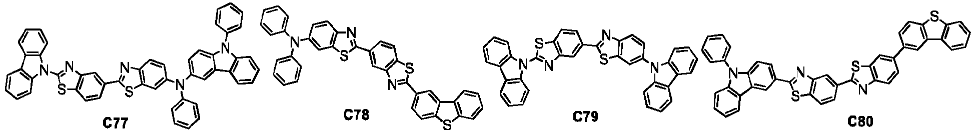

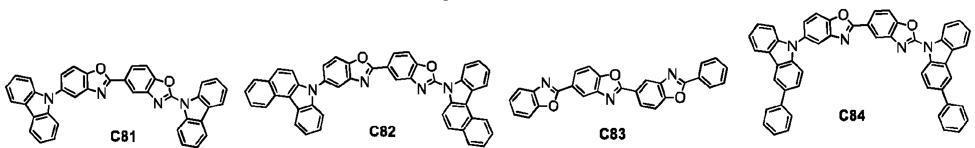

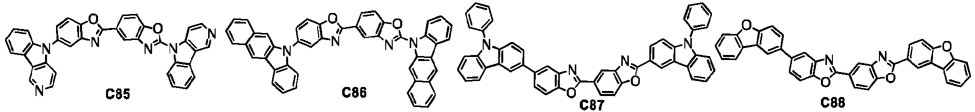

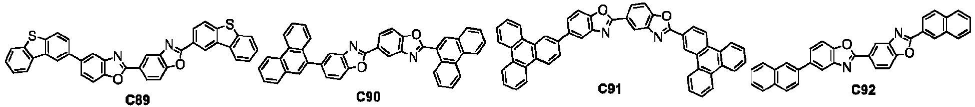

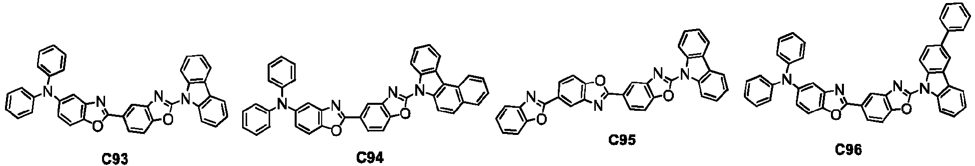

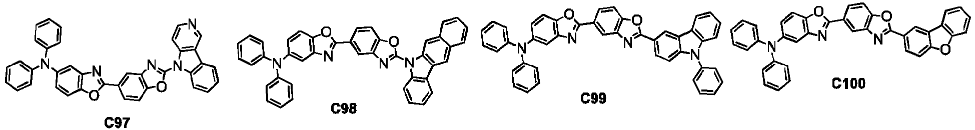

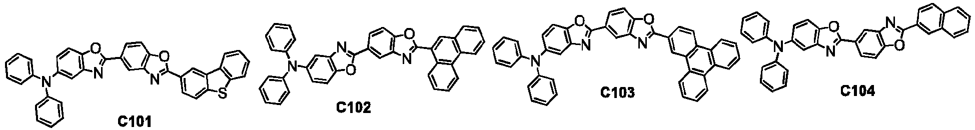

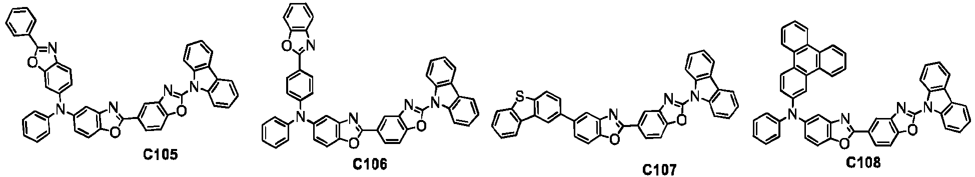

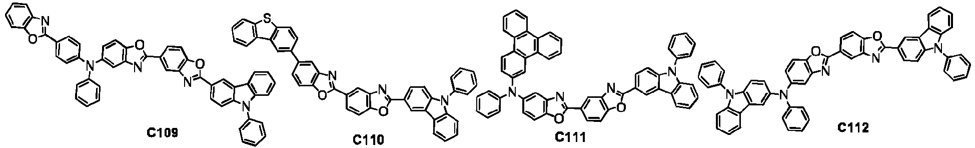

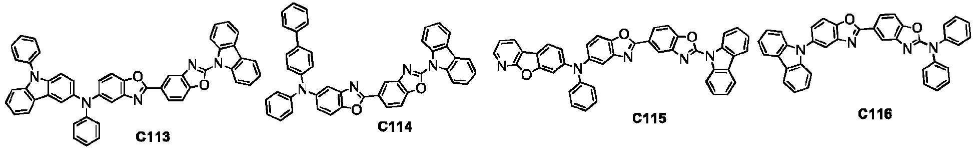

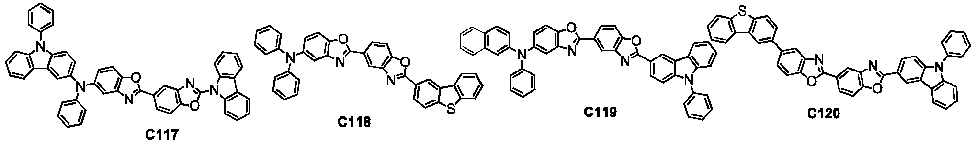

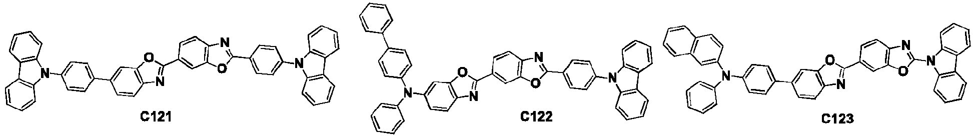

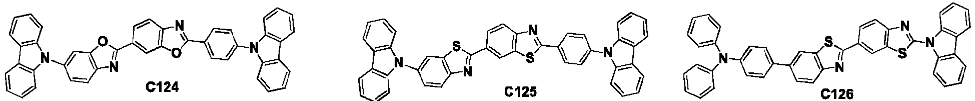

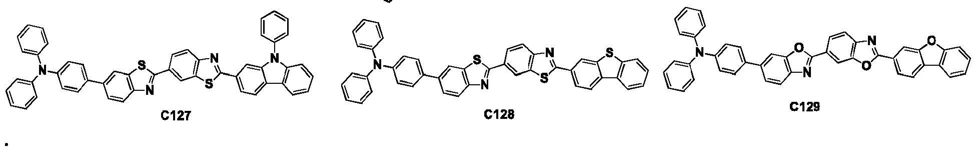

11. A light extraction layer material, wherein the light extraction layer material comprises the organic compound as claimed in claim 1.

12. The light extraction layer material of claim 11, wherein the organic compound has a structure represented by formula (2-1) or formula (2-2):

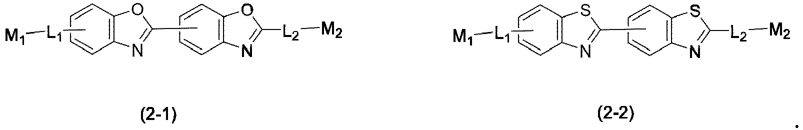

13. The light extraction layer material of claim 11, wherein L₁ and L₂ are independently selected from a single bond and a group consisting of following groups: 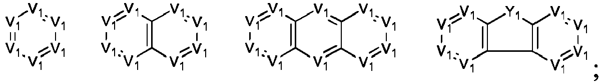 wherein,
V₁ at each occurrence is independently selected from CR₂ or N;
Y₁ is selected from NR₃, CR₄R₅, SiR₄R₅, O, S, S=O, or SO₂; and
R₂, R₃, R₄, and R₅ at each occurrence are independently selected from -H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 5 to 20 ring atoms, a substituted or unsubstituted aryloxy group having 5 to 20 ring atoms, a substituted or unsubstituted heteroaryloxy group having 5 to 20 ring atoms, or any combination of these groups.

14. The light extraction layer material of claim 11, wherein M₁ and M₂ are independently selected from a group consisting of following groups: 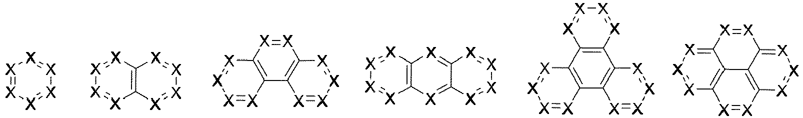 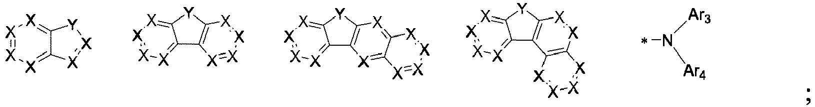 wherein,
X at each occurrence is independently selected from CR₆ or N;
Y at each occurrence is selected from NR₇, CR₈R₉, SiR₈R₉, O, S, S=O, or SO₂; and
R₆, R₇, R₈, and R₉ at each occurrence are independently selected from-H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 30 ring atoms, a substituted or unsubstituted heteroaromatic group having 5 to 30 ring atoms, a substituted or unsubstituted aryloxy group having 5 to 30 ring atoms, a substituted or unsubstituted heteroaryloxy group having 5 to 30 ring atoms, or any combination of these groups; and R₈ and R₉ form a ring or do not form a ring together;
Ar₃ and Ar₄ are independently selected from a substituted or unsubstituted aromatic group having 6 to 30 ring atoms or a substituted or unsubstituted heteroaromatic group having 5 to 30 ring atoms; and
* indicates a linking site.

15. The light extraction layer material of claim 14, wherein Ar₃ and Ar₄ are independently selected from a group consisting of following groups: 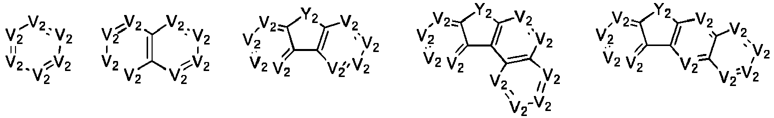 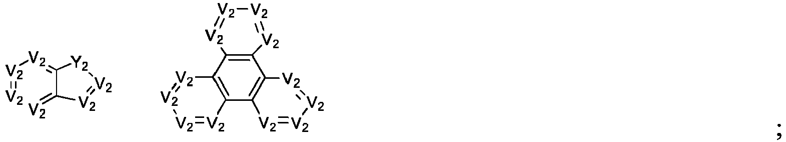 wherein,
V₂ at each occurrence is independently selected from CR₁₀ or N;
Y₂ is selected from NR₁₁, CR₁₂R₁₃, SiR₁₂R₁₃, O, S, S=O, or SO₂; and
R₁₀, R₁₁, R₁₂, and R₁₃ at each occurrence are independently selected from-H, -D, a linear alkyl group having 1 to 20 carbon atoms, a linear alkoxy group having 1 to 20 carbon atoms, a linear thioalkoxy group having 1 to 20 carbon atoms, a branched alkyl group having 3 to 20 carbon atoms, a branched alkoxy group having 3 to 20 carbon atoms, a branched thioalkoxy group having 3 to 20 carbon atoms, a cyclic alkyl group having 3 to 20 carbon atoms, a cyclic alkoxy group having 3 to 20 carbon atoms, a cyclic thioalkoxy group having 3 to 20 carbon atoms, a silyl group, a ketone group having 1 to 20 carbon atoms, an alkoxycarbonyl group having 2 to 20 carbon atoms, an aryloxycarbonyl group having 7 to 20 carbon atoms, a cyano group, an aminoformyl group, a haloformyl group, a formyl group, an isocyano group, an isocyanate group, a thiocyanate group, an isothiocyanate group, a hydroxyl group, a nitro group, an amino group, -CF₃, -Cl, -Br, -F, -I, a substituted or unsubstituted aromatic group having 6 to 20 ring atoms, a substituted or unsubstituted heteroaromatic group having 5 to 20 ring atoms, a substituted or unsubstituted aryloxy group having 5 to 20 ring atoms, a substituted or unsubstituted heteroaryloxy group having 5 to 20 ring atoms, or any combination of these groups; and R₁₂ and R₁₃ form a ring or do not form a ring together.

16. The light extraction layer material of claim 11, wherein M₁ and M₂ are independently selected from a group consisting of following groups: 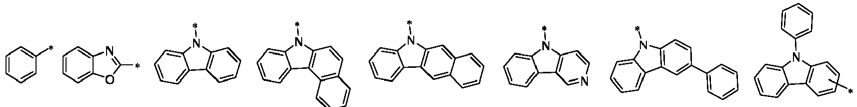 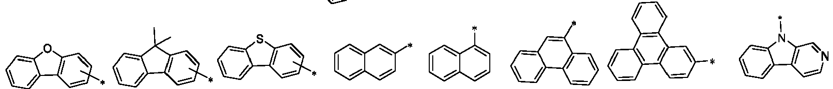 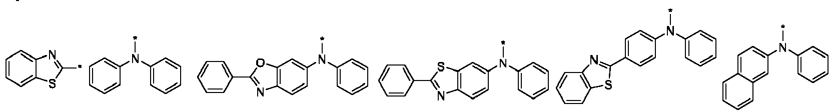 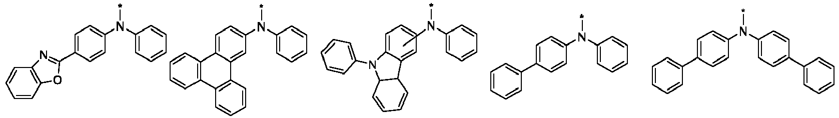 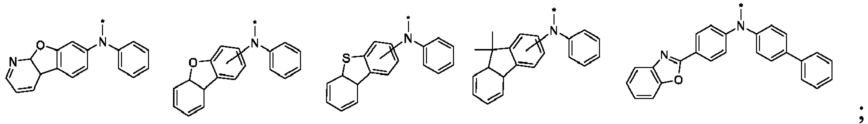 and
* indicates a linking site.

17. The light extraction layer material of claim 14, wherein the organic compound has a structure represented by any one of formulae (3-1) to (3-7):

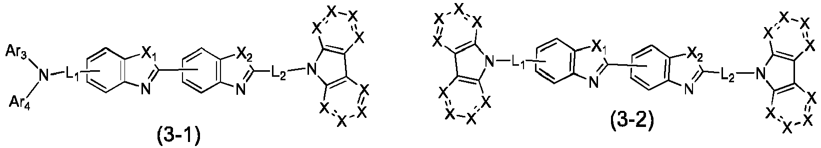

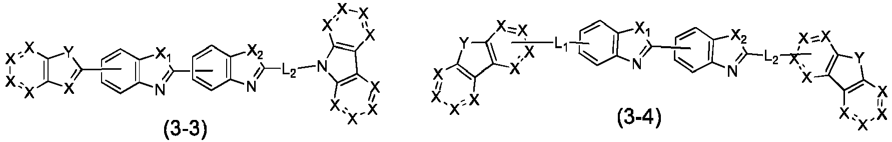

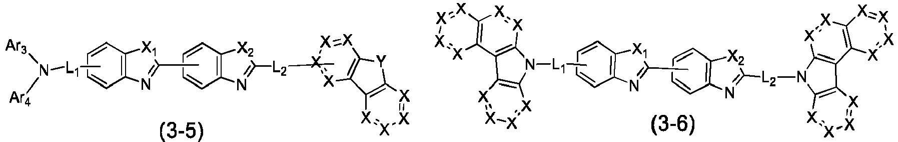

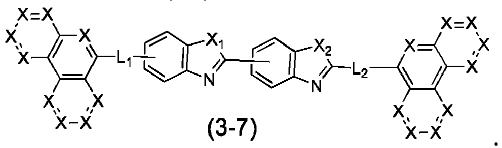

18. The light extraction layer material of claim 11, wherein the organic compound has a structure selected from a group consisting of following structures:

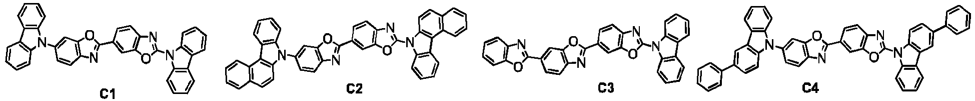

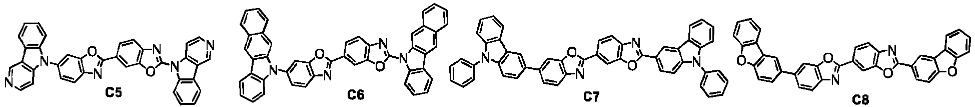

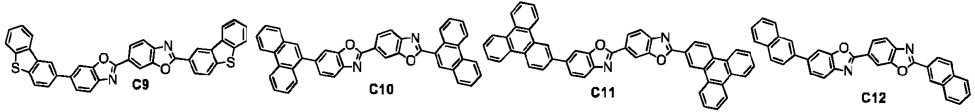

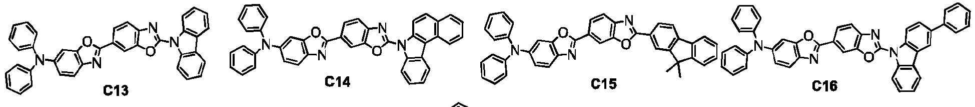

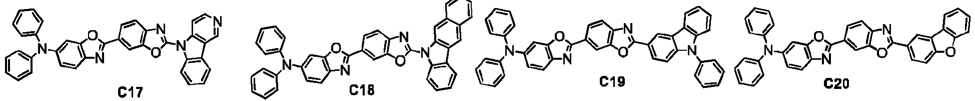

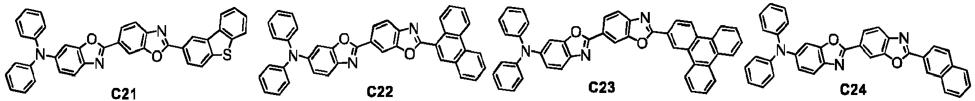

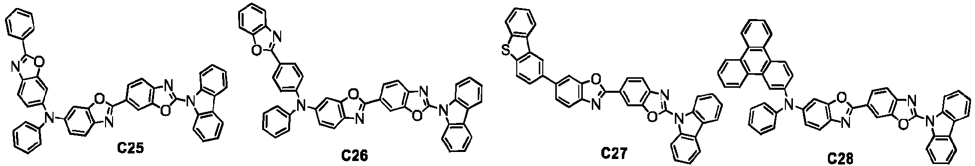

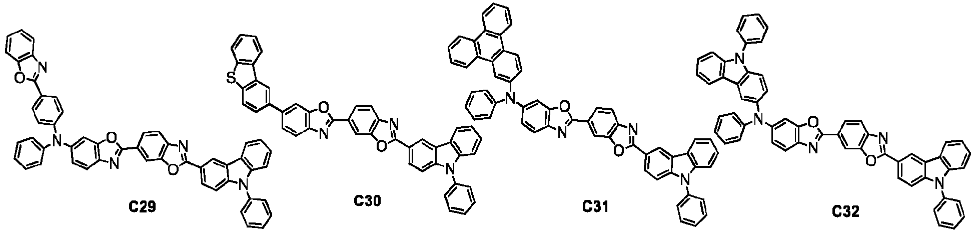

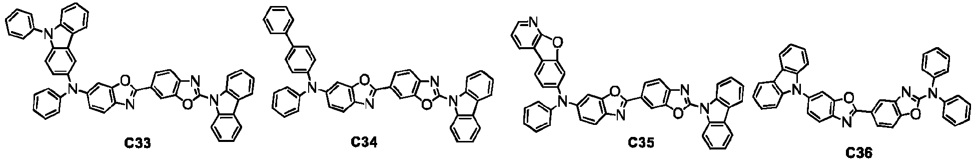

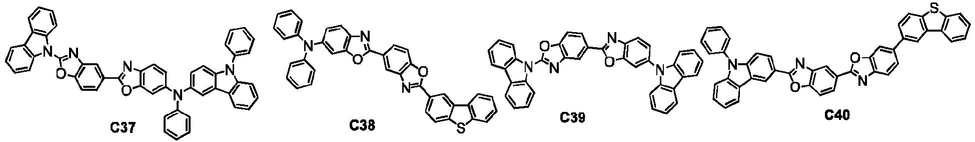

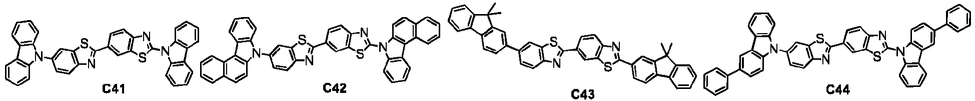

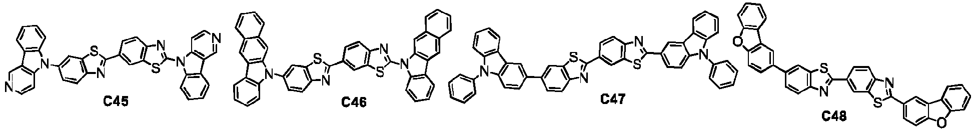

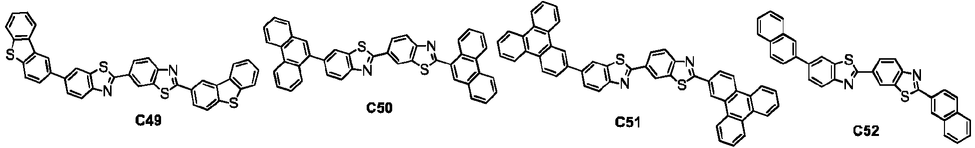

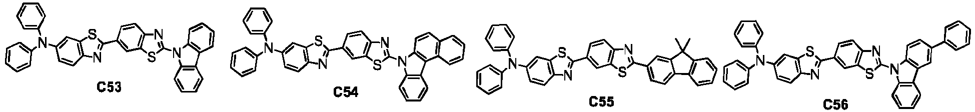

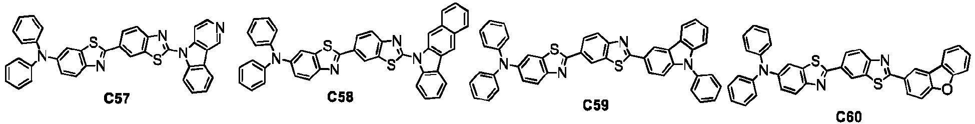

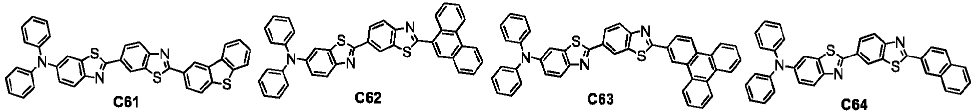

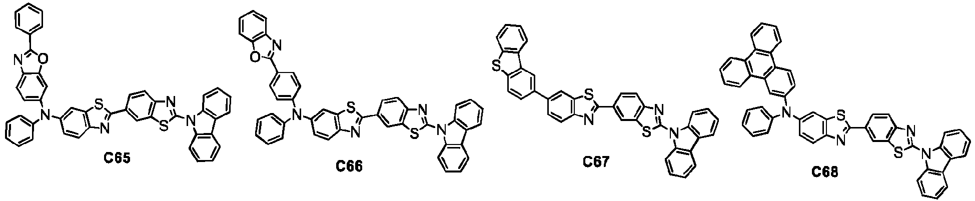

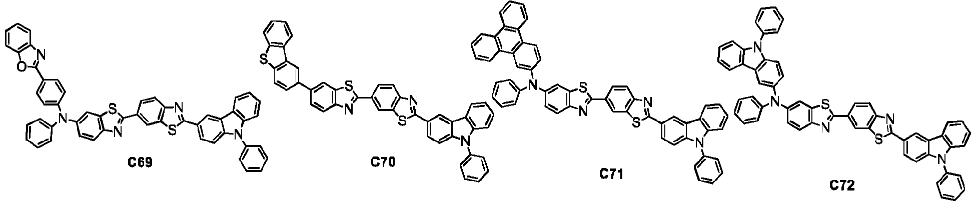

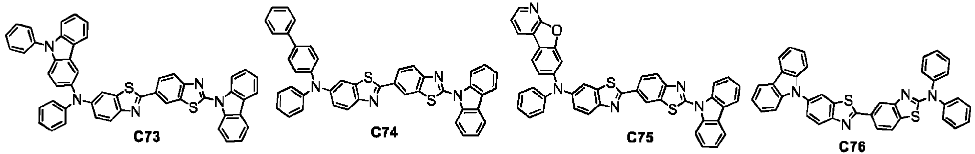

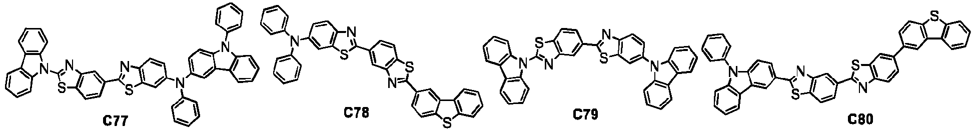

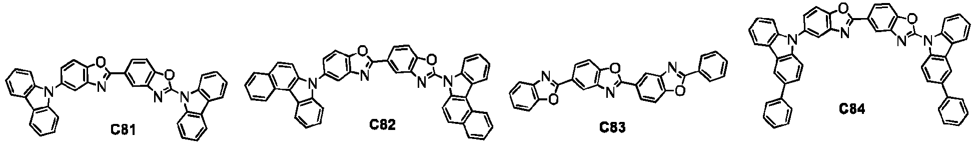

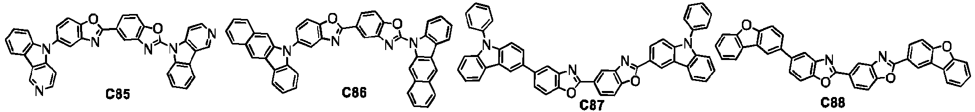

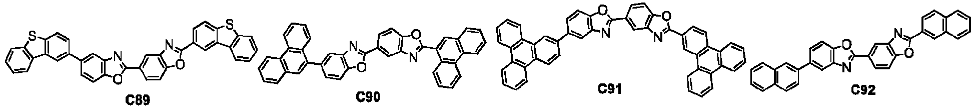

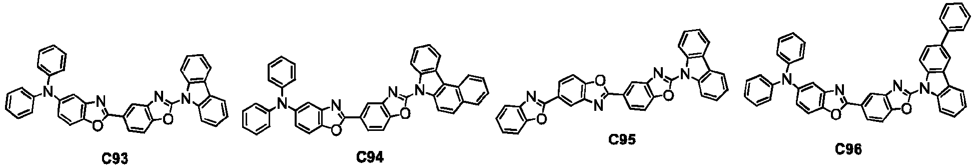

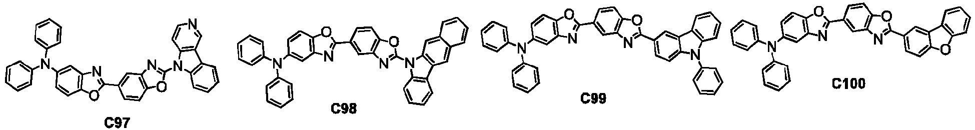

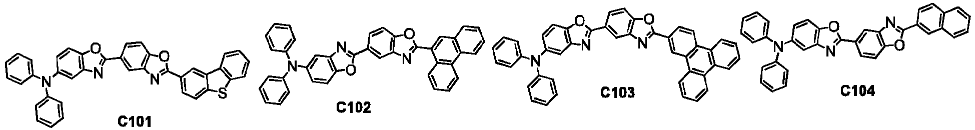

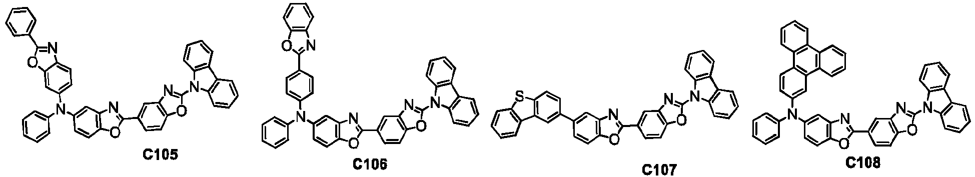

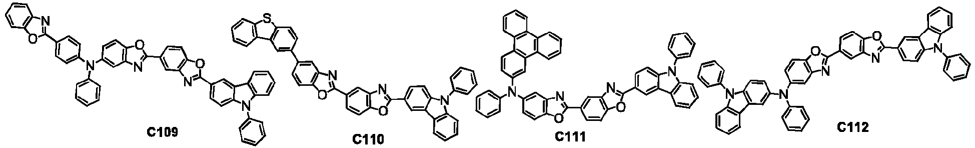

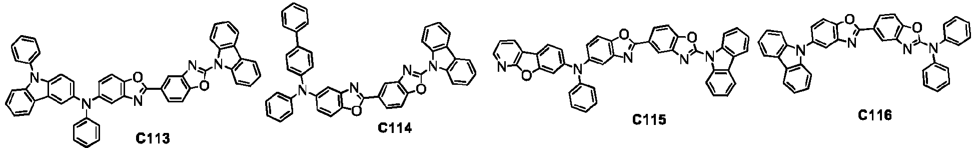

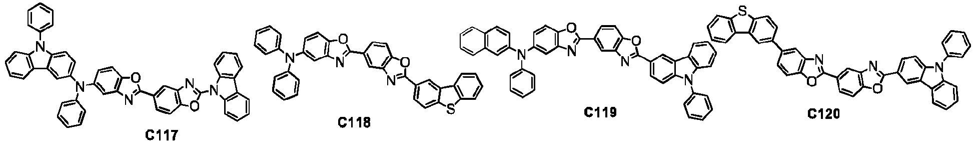

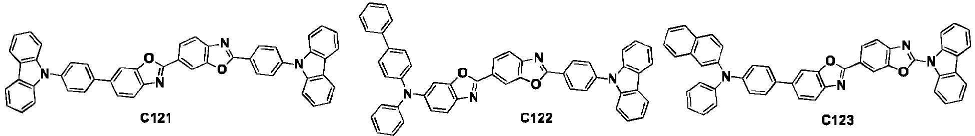

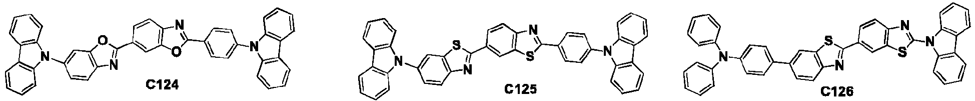

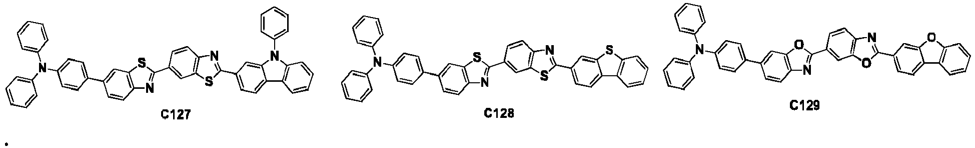

19. An organic electronic device, wherein the organic electronic device comprises the organic compound as claimed in claim 1.

20. The organic electronic device of claim 19 comprising two electrodes, one or more organic functional layers disposed between the two electrodes, and a light-extracting layer disposed at a surface of one of the two electrodes and away from the organic functional layers, wherein materials of the light-extracting layer comprises the organic compound as claimed in claim 1.
